# EUROPEAN PATENT APPLICATION

(11) **EP 1 118 856 A1**
(43) Date of publication of application: **25.07.2001**
(21) Application number: 99969788.1
(22) Date of filing: 29.09.1999
(51) Int. Cl.: G01N 27/28, G01N 31/22

(54) **SAMPLE COMPONENT ANALYSIS SYSTEM AND SENSOR CHIP AND SENSOR PACK USED FOR THE SYSTEM**

(30) Priority: 29.09.1998 JP 29150998
(71) Applicant: OMRON CORPORATION, Kyoto-shi, Kyoto 616-8025 (JP)
(72) Inventor: TOKITA, Muneo, Omron Corporation, Kyoto-shi, Kyoto 616-8025 (JP); SANO, Yoshihiko, Omron Corporation, Kyoto-shi, Kyoto 616-8025 (JP); KUKI, Kiyotsugu Omron Corporation, Kyoto-shi, Kyoto 616-8025 (JP); TANAKA, Shinya Omron Corporation, Kyoto-shi, Kyoto 616-8025 (JP)
(74) Representative: Kilian, Helmut, Dr.
(86) International application number: JP9905325
(87) International publication number: WO0019189

(57) **Abstract**

A sample ingredient analyzing system, and a sensor pack and a sensor chip usable in the system. The analyzing system has an analyzing device of a simple structure to which the sensor chip can be easily attached. The sensor pack containing the sensor chip is inserted into an opening of the analyzing device. When a slider is pushed in by the sensor pack, a supporting member rotates and a retaining member penetrates a film and plunges through a hole in the sensor chip. When the packaging material of the sensor pack is drawn out, only the sensor chip is retained by the retaining member. When a button is pressed, the supporting member rotates to release the retaining member from the retaining state, thereby enabling the sensor chip to be thrown away.

## Description

### BACKGROUND OF THE INVENTION

### 1. Field of the Invention

The present invention relates to a sample ingredient analyzing system including a sensor chip and an analyzing device, and to the sensor chip and a sensor pack used in the system. More particularly, the present invention relates to an improved system for handling a sensor chip used in an analytic process for identifying or quantifying glucose or other components in blood or a particular ingredient in industry products, food, or the like.

### 2. Description of the Related Art

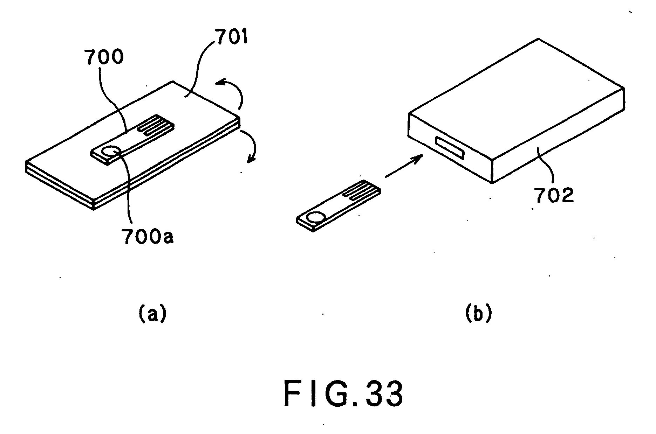

A sample ingredient analyzing system using a sensor chip as shown in Figs. 33(a) and 33(b) is known. That is, a sensor chip 700 is taken out of a packaging material 701 made of aluminum or the like and is manually set in an analyzing device 702, and a test sample is supplied to a reaction portion 700a on the sensor chip 700 by, for example, being dropped onto the reaction portion 700a to undergo analysis.

In such a system, it is necessary to take out the sensor chip 700 from the packaging material 701 each time a measurement is made. A substantially large force is required to open the packaging material 701. Also, there is a possibility of an operator inadvertently dropping the sensor chip 700 or touching the reaction portion 700a at the time of opening of the packaging material. Further, it is necessary for the sensor chip 700 with a blood sample to be carefully handled when manually detached from the analyzing device 702 after measurement. Thus, troublesome operations have been required.

As a system designed to solve this problem and to enable easier handling of a sensor chip, a system such as the one disclosed in Japanese Patent Application Laid-open No. Hei 8-262026 has been provided. In this system, a sensor pack consisting of a plurality of sensor chips enclosed in a packaging material is set in an analyzing device, and an operating lever of the analyzing device is operated to feed each sensor chip, thereby enabling use of the sensor chip.

In this case, however, the analyzing device has a complicated mechanism, which can lead to malfunction. Also, an increase in overall size and an increase in cost result.

### SUMMARY OF THE INVENTION

In view of the above-described circumstances, an object of the present invention is to provide a system using an analyzing device in which a sensor chip can be easily set, and which is simple in structure.

To achieve this object, according to a first aspect of the present invention, there is provided a sample ingredient analyzing system comprising a sensor pack formed by packing in a packaging material a sensor chip having a reaction portion for reacting with a test sample, and an analyzing device having an opening for accepting the sensor pack containing one sensor chip, and retaining means for retaining the sensor chip in the sensor pack accepted through the opening, the analyzing device analyzing an ingredient in a test sample supplied to the reaction portion by detecting a change in the reaction portion.

In this system, an operator can insert the sensor chip in the state of being packed in the sensor pack into the opening of the analyzing device without previously taking out the sensor chip from the sensor pack. After the insertion, the operator can easily set the sensor chip in the analyzing device. At the time of setting of the sensor chip, there is no risk of the operator inadvertently touching the reaction portion .

The analyzing device may be a device for quantitatively or qualitatively measuring an ingredient of a test sample by detecting a change in the reaction portion to which the test sample has been supplied. For example, the analyzing device may measure the amount of glucose in the blood from the reaction of blood and enzyme. However, the analyzing device of the present invention can measure the quantity or quality of any other material.

The above-described sensor chip may have engagement means for engagement with the retaining means.

If engagement means capable of engagement with the retaining means is provided in the sensor chip, the retaining force of the retaining member can be increased and the sensor chip can be retained more securely by the retaining means. The engagement means used for this purpose may be, for example, a recess or a projection fitted to the retaining means. However, it is not necessary to use this exclusively.

The above-described retaining means may penetrate the packaging meterial at least to reach the sensor chip.

If the retaining means is arranged to penetrate the packaging material protecting the sensor chip, it can directly contact the sensor chip to securely retain the same.

The retaining means may penetrate through the body of the sensor chip. Also, the retaining means may penetrate both through the sensor chip and through the entire packaging material. Further, the retaining means penetrating through the sensor chip may be stopped by some portion of the packaging material without penetrating the same.

The above-described sensor pack may have a holding to be held by the user.

There is a risk of causing damage to the reaction portion, or the like, to affect the analysis accuracy by applying an unnecessary force to portion including the sensor chip when handling the sensor pack holding a portion including the sensor chip. However, if the sensor pack is provided with a holding, such a risk can be prevented.

The above-described packaging material may have positioning means for positioning the sensor chip.

This positioning means facilitates relative positioning of the sensor chip and the retaining means when the sensor pack is inserted through the opening.

The above-described analyzing device may have positioning means for positioning the sensor chip when only the sensor chip is inserted through the opening.

Although use of the sensor chip by inserting it in the analyzing device in the state of being contained in the sensor pack is recommended, the user may inadvertently take out the sensor chip from the packaging material before insertion into the analyzing device. In such a case, the sensor chip can be securely held to be used for analysis if the sensor chip positioning means is provided. Thus, waste of the sensor chip is prevented.

The above-described analyzing device may have retention undoing means for undoing the retention continued by the retaining means.

After use, the sensor chip has a test sample attached thereon. Some test sample such as blood should not be touched thoughtlessly. The analyzing system may be arranged to enable detachment of the sensor chip in such a manner that retention undoing means, such as a lever or a button provided in the analyzing device, is operated to release the sensor chip from the state of being retained by the retaining means. By operating the retention undoing means, the user can throw away the sensor chip without touching the test sample or the sensor chip.

The above-described analyzing device may have state changing means for changing the state of the retaining means between a state of receding from the sensor pack and a state of retaining the sensor chip. The state changing means may be arranged to change the state of the retaining means so that the retaining means is in the receding state when the sensor pack is inserted, and so that the retaining means is in the retaining state after the completion of insertion of the sensor pack.

This arrangement is intended to prevent the retaining means from impeding insertion of the sensor pack into the opening of the analyzing device, and to securely retain the sensor chip after the completion of insertion of the sensor pack.

The above-described state changing means may be operated by a movable member which is moved by insertion of the sensor pack.

If the state changing means is operated in this manner, the operator may insert the sensor pack without performing any special operations for retaining the sensor chip in the process of inserting the sensor pack and retaining the sensor chip.

Also, the above-described analyzing device may have a power supply switch operated by the movement of the movable member.

The power supply switch can be operated by insertion and detachment of the sensor pack. It is not necessary for the operator to be conscious of the on/off state of the power supply. This arrangement is also effective in reducing the possibility of omission of turning on or off the power supply.

The above-described analyzing device may alternatively have a power supply switch capable of turning on and off the power supply to the analyzing device by being linked to the two states of the retaining means.

The power supply switch can be operated by insertion and detachment of the sensor pack. It is not necessary for the operator to be conscious of the on/off state of the power. This arrangement is also effective in reducing the possibility of omission of turning on or off the power supply.

The above-described analyzing device may have reaction information acquisition means for obtaining information on reaction at the reaction portion from the sensor chip. Also, the analyzing device may position the reaction information acquisition means on the sensor chip by retaining the sensor chip by the retaining means.

In this manner, the reaction information acquisition means can be positioned simultaneously with retaining of the sensor chip by the retaining means to obtain reaction information such as information of a change in the reaction portion. Thus, the analyzing device can be made easier to operate.

The reaction information acquisition means may be one capable of electrically contacting the sensor chip and obtaining information such as a change in the reaction portion as an electrical signal, or one capable of optically reading a change in the reaction portion as, for example, a change in color without contacting the sensor chip. However, other various means are also conceivable as usable means capable of obtaining information on a change in the reaction portion in accordance with the present invention.

In the above-described sample ingredient analyzing system, when the packaging material is removed from the opening while the sensor chip is retained by the retaining means, the sensor chip may be taken out from the packaging material in such a manner that the sensor chip is brought into contact with the packaging material to tear the packaging material, and the packaging material may have a force receiving portion provided at a position at which the sensor chip is brought into contact with the packaging material, a force applied by the sensor chip being concentrated at the force receiving portion.

If the sensor chip can tear the packaging material by itself as described above to exit from the packaging material, it is not necessary to provide a special member for taking out the sensor chip. Also, the force applied from the packaging material can be concentrated at the force receiving portion, and the packaging material can rupture easily at the force receiving portion. As a result, the sensor chip can be easily taken out by a small force.

Also, a portion of the sensor chip remote from the reaction portion may be taken out first from the packing material.

If the sensor chip is taken out in this manner, the reaction portion can be protected from an impact or contact when the sensor chip is taken out from the film.

The above-described analyzing device may have reaction information acquisition means for obtaining information on reaction at the reaction portion from the sensor chip, and the reaction information acquisition means may be provided in the retaining means.

This arrangement is effective in simplifying the construction of the analyzing device and in reducing the number of component parts.

The above-described packaging material may have a penetrable portion through which the retaining means can penetrate, and a penetration stop portion which stops the penetrating action of the retaining means. The above-described analyzing device may have reaction information acquisition means for obtaining information on reaction at the reaction portion by being brought into contact with the sensor chip, and state changing means for changing the state of the reaction information acquisition means between a first state of being spaced apart from the sensor pack or loosely contacting the sensor pack and a second state of contacting the sensor chip. The state changing means may set the reaction information acquisition means in the first state when the retaining means penetrates the penetrable portion to retain the sensor chip, and similarly may set the reaction information acquisition means in the second state when the packaging material is removed from the opening, and when the retaining means is retaining only the sensor chip.

In the analyzing device arranged in this manner, the reaction information acquisition means is spaced apart from the sensor pack or loosely contacts the sensor pack before the packaging material is removed from the opening. Therefore, some fat or oil or other contaminants attached to the sensor pack are prevented from being attached to the reaction information acquisition means, and wear of the reaction information acquisition means can also be prevented, thus preventing degradation of the reaction information acquisition means and reducing the resistance to the movement of the packaging material when the packaging material is drawn out.

The above-described sensor pack may contain a desiccant.

The desiccant contained in the sensor pack can absorb moisture in air left in the pack when the sensor chip is packed in the packaging material, thereby maintaining the quality of the sensor chip. Even if some moisture enters the completed pack by passing through the packaging material, it can be absorbed by the desiccant. The desiccant may be separately provided and accommodated in an accommodation portion formed for the desiccant. Also, if the packaging material is made of a resin or the like, the desiccant may be mixed in the resin material to be contained in the packaging material.

The above-described sensor pack may have a holding to be held by a user, and a desiccant accommodation portion for accommodating the desiccant may be provided in the holding.

If a desiccant accommodation portion is formed, the holding is shaped by forming a projection or a recess, so that the holding becomes easier to be hold. The combination of the holding and the desiccant accommodation portion is effective in improving the efficiency of space utilization and in limiting the size of the sensor pack.

In the above-described sample ingredient analyzing system, a predetermined orientation of the sensor pack with respect to the direction of insertion into the opening of the analyzing device may be prescribed, and a cross-sectional shape of the sensor pack as viewed in the direction of insertion when the sensor pack has an orientation different from the predetermined orientation may be made different from a cross-sectional shape of the opening as viewed in the direction of insertion.

This is intended to prevent insertion of the sensor pack in the state of having an orientation different from the predetermined orientation.

The above-described sensor chip may have the shape of a generally flat block, and each of the sensor pack and the opening may have a shape exhibiting an asymmetry on the opposite sides of two surfaces of the sensor chip.

If the sensor pack and the opening are formed in this manner, and if the sensor chip is a planar sensor or the like having the shape of a generally flat block, the sensor chip can be prevented from being inserted the wrong side up.

Also, the above-described sensor chip may have the shape of a generally flat block, and each of the sensor pack and the opening has a shape asymmetric as seen in opposite directions along a surface of the sensor chip.

If the sensor pack and the opening are formed in this manner, and if the sensor chip is a planar sensor or the like having the shape of a generally flat block, the sensor chip can be prevented from being inserted the wrong side up, or insertion of the wrong end of the sensor chip can be prevented.

Also, a predetermined orientation of the sensor pack with respect to the direction of insertion into the opening of the analyzing device may be prescribed, and a portion of the sensor pack on one side in the direction of insertion along the predetermined orientation and another portion of the sensor pack on the opposite side may be made different in shape from each other.

This is also intended to prevent insertion of the wrong end of the sensor pack.

The above-described sample ingredient analyzing system may further include inserted state detection means having a detecting portion provided in the analyzing device and a portion to be detected provided in the sensor pack at a predetermined position.

This means makes it possible to determine the inserted state, i.e., whether the sensor pack is correctly inserted from a check as to whether the portion to be detected provided at the predetermined position in the sensor pack has been detected with the detecting portion, thereby enabling prevention of insertion of the wrong end or in the state of wrongly oriented. Since a wrongly inserted state can also be detected, waste of the sensor pack can be prevented. Notifying means for notifying the result of detection performed by the inserted state detection means through letters, speech, signal of light or the like provided for user's convenience.

The above-described analyzing device may have first reaction information acquisition means for obtaining information on reaction at the reaction portion from the sensor chip when the sensor chip is inserted in the state of having the predetermined orientation with respect to the opening, and second reaction information acquisition means for obtaining information on reaction at the reaction portion from the sensor chip when the sensor chip is inserted in the state of having an orientation different from the predetermined orientation.

In the analyzing device using these means, even if the sensor pack or the sensor chip is inserted in the state of being wrongly oriented, information on reaction can be obtained by the second reaction information acquisition means to be analyzed, thus improving the operability. The second reaction information acquisition means may be provided to enable acquisition of reaction information with respect to insertion in the state of having a supposed orientation according to the shape of the sensor pack or the sensor chip. Two or more second reaction information acquisition means may be provided.

The sample ingredient analyzing system may include information holding means for holding information on the sensor chip, the information holding means being provided on at least one of the sensor pack and the sensor chip, and information recognition means for recognizing information held by the information holding means, information recognition means being provided in the analyzing device.

Generally, a plurality of the above-described sensor chips vary in characteristics. If the fluctuation with respect to one characteristic is large, there is a need for correcting the characteristic. Characteristic correction in such a case is ordinarily performed by, for example, the method of inserting a correction chip in the analyzing device or the method of inputting a correction value. However, these method require a complicated correction procedure, and a possibility of an input error or omission of correction is also a consideration. Then, a method may be used in which, as described above, information holding means is provided on at least one of the sensor pack and the sensor chip, necessary information such as a lot number or a correction value is held in the information holding means, and this information is recognized by information recognition means in the analyzing device, thereby eliminating the need for a preliminary setting process including inserting a correction chip in the analyzing device and inputting a correction value. The possibility of errors is also eliminated. Since each sensor pack is separately inserted in the analyzing device, a lot number, a correction value, the date of manufacture, and the like, may be held on each sensor pack. If information holding means is provided in the sensor pack, it is possible to measure the characteristics of the sensor chip and determine a correction value after manufacture of the sensor pack. This means that sensor packs can be manufactured more easily.

The sample ingredient analyzing system may include insertion orientation determination means for making a determination as to whether the orientation of the sensor chip with respect to the direction of insertion is correct by checking whether information from the information holding means can be recognized by the informaition recognition means.

In this manner, the information holding means and the information recognition means can also be used for determination as to whether the orientation of the sensor chip with respect to the direction of insertion is correct. Notifying means or reinsertion instruction means may also be provided. When the insertion orientation determination means determines an error in setting the orientation with respect to the direction of insertion, the notifying means or reinsertion instruction means notifies this result or instructs the operator to reinsert the sensor chip. If these means are provided, the analyzing device becomes more convenient.

The above-described analyzing device may have opening forming means for forming an opening in the packagin material of the sensor pack.

The opening forming means can form an opening in the sensor pack through which the sensor chip is taken out. The force required to taken out the sensor chip is thus reduced, so that the operability of the analyzing device is improved. Rupturing means such as a cutter may be used as this opening forming means.

The analyzing device may have speech generation means.

The speech generation means can provide information on use of the analyzing device, an analysis result, an error, remeasurement, and the like, thereby enabling even a weak-sighted person to operate the analyzing device.

According to a second aspect of the present invention, there is provided a sensor chip for use in a sample ingredient analyzing system having a sensor pack formed by packing in a packaging material a sensor chip having a reaction portion for reacting with a test sample, and an analyzing device having an opening for accepting the sensor pack containing one sensor chip, and retaining means for retaining the sensor chip in the sensor pack accepted through the opening, the analyzing device analyzing an ingredient in the test sample supplied to the reaction portion by detecting a change in the reaction portion, the sensor chip comprising engagement means for engagement with the retaining means of the analyzing device.

A sensor chip capable of being securely retained by receiving an increased retaining force from the retaining means can be provided if such engagement means for engagement with the retaining means is used.

According to a third aspect of the present invention, there is provided a sensor pack for use in a sample ingredient analyzing system having the sensor pack formed by packing in a packaging material a sensor chip having a reaction portion for reacting with a test sample, and an analyzing device for analyzing an ingredient in a test sample supplied to the reaction portion by detecting a change in the reaction portion, the sensor pack comprising a holding to be held by a user.

The provision in the sensor pack of a holding to be held by a user is effective in reducing the risk of causing damage to the reaction portion, etc., to affect the analysis accuracy by applying an unnecessary force to the sensor chip accommodation portion during handling of the sensor pack by holding that portion.

According to a fourth aspect of the present invention, there is provided a sensor pack for use in a sample ingredient analyzing system having the sensor pack formed by packing in a packaging material a sensor chip having a reaction portion for reacting with a test sample, and an analyzing device for analyzing an ingredient in a test sample supplied to the reaction portion by detecting a change in the reaction portion, the sensor pack comprising positioning means for positioning the sensor chip, the positioning means provided in the packaging material.

In this manner, a sensor pack capable of easily performing relative positioning of the sensor chip and the retaining means when inserted through the opening can be provided.

According to a fifth aspect of the present invention, there is provided a sensor pack for use in a sample ingredient analyzing system having the sensor pack formed by packing in a sensor chip having a reaction portion for reacting with a test sample, and an analyzing device for analyzing an ingredient in a test sample supplied to the reaction portion by detecting a change in the reaction portion, in which system, when the packaging material is removed from an opening of the analyzing device while the sensor chip is retained by retaining means, the sensor chip is taken out from the packaging material in such a manner that the sensor chip is brought into contact with the packaging material to tear the packaging material, the sensor pack comprising a force receiving portion provided in the packaging material at a position at which the sensor chip is brought into contact with the packaging material, a force applied by the sensor chip being concentrated at the force receiving portion.

If the sensor chip can tear the packaging material by itself as described above to exit from the packaging material, it is not necessary to provide a special member for taking out the sensor chip. Also, the force applied by the sensor chip can be concentrated at the force receiving portion, and the packaging material can rupture easily at the force receiving portion. Thus, a sensor pack capable of easily taking out the sensor chip by a small force can be provided.

According to a sixth aspect of the present invention, there is provided a sensor pack for use in a sample ingredient analyzing system having the sensor pack formed by packing in a packaging material a sensor chip having a reaction portion for reacting with a test sample, and an analyzing device for analyzing an ingredient in a test sample supplied to the reaction portion by detecting a change in the reaction portion, the sensor pack comprising a desiccant.

A sensor pack capable of maintaining the quality of the sensor chip can be provided by using a desiccant which can absorb moisture in the air left in the pack when the sensor chip is packed in the packaging material. Even if some moisture enters the completed pack by passing through the packaging material, it can be absorbed by the desiccant. The desiccant may be separately provided and accommodated in an accommodation portion formed for the desiccant. Also, if the packaging material is made of a resin or the like, a desiccant may be mixed in the resin material to be contained in the packaging material.

The above-described sensor chip may have a holding to be held by a user, and a desiccant accommodation portion for accommodating the desiccant may be provided in the holding.

If a desiccant accommodation portion is formed, the holding is shaped by forming a projection or a recess. Thus, a sensor pack which is easily held can be provided. By providing the holding which can also serve as the desiccant accommodation portion, it is possible to improve the efficiency of space utilization, and thereby to limit the size of the sensor pack.

According to a seventh aspect of the present invention, there is provided a sensor pack for use in a sample ingredient analyzing system having the sensor pack formed by packing in a packaging material a sensor chip having a reaction portion for reacting with a test sample, and an analyzing device for analyzing an ingredient in a test sample supplied to the reaction portion by detecting a change in the reaction portion. In this sensor pack, a predetermined orientation of the sensor pack is prescribed with respect to the direction of insertion into an opening of the analyzing device, and a cross-sectional shape of the sensor pack as viewed in the direction of insertion when the sensor pack has an orientation different from the predetermined orientation is different from a cross-sectional shape of the opening of the analyzing device as viewed in the direction of insertion.

In this manner, a sensor pack which can easily prevent insertion in a state of having an orientation different from the predetermined orientation can be provided.

According to an eighth aspect of the present invention, there is provided a sensor pack for use in a sample ingredient analyzing system having the sensor pack formed by packing in a packaging material a sensor chip having a reaction portion for reacting with a test sample, and an analyzing device for analyzing an ingredient in a test sample supplied to the reaction portion by detecting a change in the reaction portion. In this sensor pack, a portion of the sensor pack on one side in the direction of insertion and another portion of the sensor pack on the opposite side are made different in shape from each other.

In this manner, a sensor pack which can easily prevent insertion of the wrong end can be provided.

According to a ninth aspect of the present invention, there is provided a sensor pack for use in a sample ingredient analyzing system having the sensor pack formed by packing in a packaging material a sensor chip having a reaction portion for reacting with a test sample, and an analyzing device for analyzing an ingredient in a test sample supplied to the reaction portion by detecting a change in the reaction portion, the analyzing device having information recognition means. This sensor pack has information holding means for holding information recognizable by the information recognition means.

If, as described above, information holding means is provided in at least one of the sensor pack and the sensor chip, necessary information such as a lot number and a correction value is held in the information holding means, and this information is recognized by information recognition means in the analyzing device, a setting process necessary for correction of a chip characteristic can be simplified, and the possibility of errors is eliminated. If information holding means is provided in the sensor pack, it is possible to measure the characteristics of the sensor chip and determine a correction value after manufacture of the sensor pack. This means that sensor packs can be manufactured more easily.

### BRIEF DESCRIPTION OF THE DRAWINGS

Figs. 1(a), 1(b), and 1(c) are diagrams showing an analyzing device and a sensor pack in a first embodiment of the present invention;
Fig. 2 is a diagram showing a sensor chip and a packaging material in the first embodiment of the present invention;
Figs. 3(a) through 3(d) are diagrams for explaining the sensor chip retaining operation of the analyzing device in the first embodiment of the present invention;
Fig. 4 is a diagram showing an analyzing device and a sensor pack in a second embodiment of the present invention;
Figs. 5(a) through 5(d) are diagrams showing an analyzing device and a sensor pack in a third embodiment of the present invention;
Figs. 6(a) and 6(b) are diagrams showing an analyzing device and a sensor pack in a fourth embodiment of the present invention;
Fig. 7 is a diagram showing an analyzing device and a sensor pack in a fifth embodiment of the present invention;
Fig. 8 is a diagram showing essential portions of an analyzing device and a sensor pack in a sixth embodiment of the present invention;
Fig. 9 is a diagram showing essential portions of an analyzing device and a sensor pack in a seventh embodiment of the present invention;
Figs. 10(a) and 10(b) are diagrams showing a sensor chip and a retaining members in an eighth embodiment of the present invention;
Fig. 11 is a diagram showing an example of modification of the eighth embodiment of the present invention;
Figs. 12(a), 12(b), and 12(c) are diagrams showing a sensor chip and a retaining member in a ninth embodiment of the present invention;
Fig. 13 is a diagram showing a sensor pack in a tenth embodiment of the present invention;
Figs. 14(a) and 14(b) are diagrams showing essential portions of an analyzing device in an eleventh embodiment of the present invention;
Figs. 15(a), 15(b), and 15(c) are diagrams for explaining the sensor chip taking out operation of an analyzing device in a twelfth embodiment of the present invention;
Figs. 16(a), 16(b), and 16(c) are diagrams showing essential portions of an analyzing device and a sensor chip in a thirteenth embodiment of the present invention;
Fig. 17(a) is a diagram showing an analyzing device and a sensor pack in a fourteenth embodiment of the present invention;
Fig. 17(b) is a diagram showing the analyzing device and the sensor pack in a used state in the fourteenth embodiment of the present invention;
Figs. 18(a) and 18(b) are cross-sectional views of essential portions of an analyzing device and a sensor pack in a fifteenth embodiment of the present invention;
Fig. 19 is a cross-sectional view of an example of modification of the fifteenth embodiment of the present invention;
Fig. 20(a) and 20(b) are a top view and a cross-sectional view, respectively, of a sensor pack constituting a sample ingredient analyzing system in a sixteenth embodiment of the present invention;
Figs. 21(a), 21(b), and 21(c) are diagrams showing an analyzing device and a sensor pack constituting a sample ingredient analyzing system in a seventeenth embodiment of the present invention;
Figs. 22(a), 22(b), and 22(c) are diagrams showing an analyzing device and a sensor pack constituting a sample ingredient analyzing system in an eighteenth embodiment of the present invention;
Fig. 23 is a diagram showing a sensor pack constituting a sample ingredient analyzing system in a nineteenth embodiment of the present invention;
Fig. 24 is a schematic perspective view of a first modification of the nineteenth embodiment of the present invention;
Figs. 25(a) and 25(b) are schematic perspective views of a second modification of the nineteenth embodiment of the present invention;
Fig. 26(a) is a perspective view of an analyzing device and a sensor pack in a twentieth embodiment of the present invention;
Fig. 26(b) is a cross-sectional view of the analyzing device and the sensor pack in the twentieth embodiment of the present invention;
Figs. 27(a) and 27(b) are diagrams showing an example of modification of the twentieth embodiment of the present invention;
Fig. 28 is a cross-sectional view of an analyzing device and a sensor chip in a twenty-first embodiment of the present invention;
Figs. 29(a) and 29(b) are diagrams schematically showing the construction of related portions of an analyzing device and a sensor chip in a twenty-second embodiment of the present invention and a modification of the same;
Figs. 30(a) through 30(h) are diagrams schematically showing the construction of related portions of an analyzing device and a sensor pack in a twenty-third embodiment of the present invention;
Figs. 31(a) through 31(f) are cross-sectional views of an analyzing device and a sensor pack in a twenty-fourth embodiment of the present invention;
Fig. 32 is a block diagram of an analyzing device in a twenty-fifth embodiment of the present invention; and
Fig. 33(a) and 33(b) are diagrams showing a sensor pack and an analyzing device in the conventional art.

### DESCRIPTION OF THE PREFERRED EMBODIMENTS

Embodiments of the present invention will be described with reference to the accompanying drawings.

### (First Embodiment)

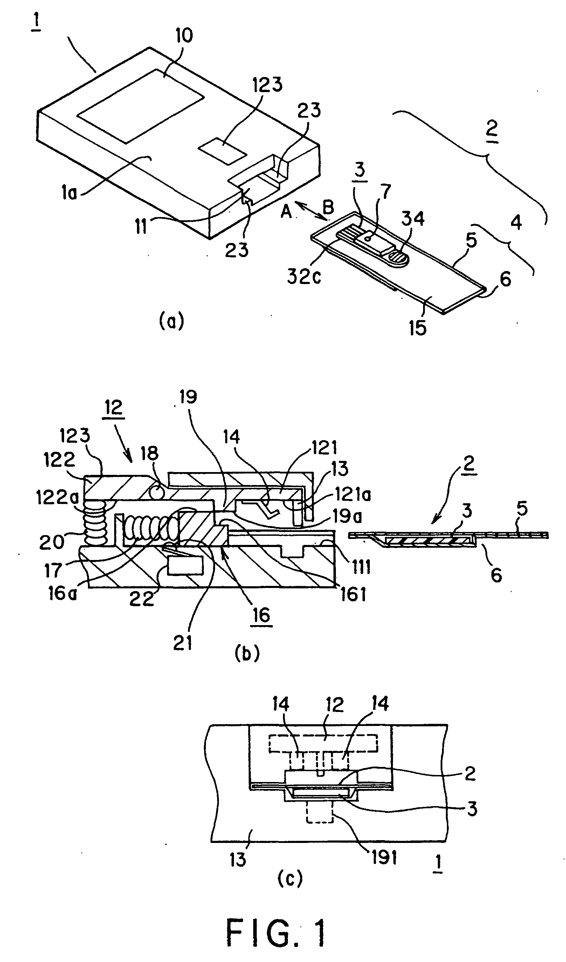

Fig. 1(a) is a perspective view of the whole of an analyzing device 1 and a sensor pack 2, which constitute a sample ingredient analyzing system according to an embodiment of the present invention. Fig. 1(b) is a cross-sectional view of the analyzing device 1 and the sensor pack 2 taken along a line passing through the center of an opening 11 in the longitudinal direction (in the direction of insertion of the sensor pack) to schematically show the construction of essential portions of the analyzing device 1 and the construction of the sensor pack 2. Fig. 1(c) is a view of the analyzing device 1 seen from the opening 11 side.

The sensor pack 2 has a sensor chip 3 accommodated in a packaging material 4.

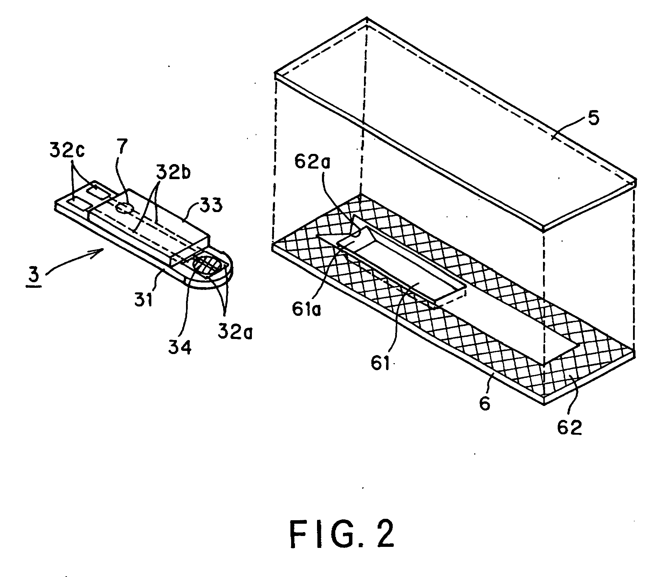

Fig. 2(a) is a perspective view of the entire structure of the sensor chip, and Fig. 2(b) is an exploded perspective view of the structure of the packaging material.

The sensor chip 3 is constructed as described below. Carbon paste is printed by screen printing on a base plate 31 which is an insulating film formed of polyethylene terephthalate, for example. The carbon paste is dried by being heated or irradiated with ultraviolet rays to be hardened. A pair of reaction electrode portions 32a,32a, a pair of lead portions 32b,32b and a pair of terminal electrode portions 32c,32c are thereby formed. An insulating film (not shown) and a cover film 33 formed of polyethylene terephthalate are formed over the lead portions 32b,32b. A reagent layer 34 is formed as a reaction portion on the reaction electrode portions 32a,32a by spreading and drying a reagent containing an enzyme. The shape and the structure of the sensor chip 3 and the method of manufacturing the sensor chip 3 are not limited to those described above or illustrated in the figures.

The packaging material 4 is constituted of a film 5 made of metal such as aluminum or plastic, and a base 6 formed of a plastic sheet or a sheet made of metal such as aluminum. An indented portion (positioning means) 61 for accommodating and positioning the sensor chip 3 is formed in the base 6. The base 6 has a holdable extension 15 as extended in the direction opposite to the direction of insertion into the analyzing device so as to be easily held between operator's fingers. The film 5 and the base 6 are bonded to each other by being thermowelded in a sealing manner. In a bonded portion 62 indicated by hatching, a bonding surface portion on the analyzing device insertion side, adjacent to a package region through which the sensor chip 3 will be taken out, is specially shaped by being notched so as to leave an angular portion, thereby forming a force-receiving portion 62a at which a force from the sensor chip 3 is concentrated. The contact area when the film 5 is torn is thus reduced, so that the sensor chip 3 can be easily taken out by a small force. Since the reagent layer 34 and the reaction electrode portions 32a of the sensor chip 3 are provided on the side opposite from the end of the sensor chip 3 facing in the direction of insertion, thereby being prevented from an impact or contact when the sensor chip 3 is taken out by tearing the film. Also, the sensor pack 2 is formed so as to have the holdable extension to be held between operator's fingers. Therefore there is substantially no risk of the operator holding a portion containing the sensor chip 3 and applying an unnecessary force to cause damage to the reagent layer or other portions, which results in a reduction in analysis accuracy. Thus, accurate analysis can be ensured. In this embodiment, the sensor pack is formed into generally a rectangular shape. However, the sensor pack may be have a different shape such that the area of the holdable extension is increased to make the extension much easier to be held. The shape of the sensor pack is not limited to such a shape.

The opening 11 formed in the analyzing device 1 has a size large enough to enable insertion of the sensor pack 2. A slider (movable member) 16 is provided at an inner portion of the opening 11. The slider 16 can move on a bottom surface 111 in the direction of insertion of the sensor pack 2. A spring 17 is provided on the side of the slider 16 opposite from the opening 11. When the slider 16 is moved from an initial position in Fig. 1(b) the direction of moving away from the opening 11, the spring 17 urges and presses the slider 16 toward the opening 11. A supporting member 12 is provided swingably on a shaft 18. The supporting member 12 includes a retaining member (retaining means) 13 in the form of a plate or a rod downwardly extending from a lower surface 121a of an opening 11-side arm portion 121 of the supporting member 12 at a center in the width direction (parallel to the direction of projection of Fig. 1(b)). The supporting member 12 has two connection electrodes (reaction information acquisition means) 14 arranged in the width direction on the shaft 18 side of the retaining member 13. Further, the supporting member 12 has a projection 19 having generally a rectangular cross section and located between the connection electrodes 14 and the shaft 18. A spring 20 is provided between the bottom surface 111 and a lower surface 122a of an arm portion 122 of the supporting member 12 opposite from the opening 11 to upwardly urge and press the lower surface 122a of the arm portion 122. The opening 11-side arm portion 121 of the supporting member 12 is thus urged downwardly. A stepped portion 161 is formed in an opening-side end portion of an upper surface 16a of the slider 16 so as to be able to fittingly receive the projection 19 of the supporting member 12. When the slider 16 is in the initial position as shown in Fig. 1(b), a lower surface 19a of the projection 19 and the upper surface 16a of the slider 16 contact each other to limit the clockwise rotation of the arm portion 121. In this state, therefore, the retaining member 13 does not contact the sensor pack 2 when the sensor pack 2 is inserted through the opening 11. An actuator 21 is projecting through the bottom surface 111 at the back of the slider 16 as seen from the opening 11. A switch 22 is provided which detects a movement of the actuator 21 when the slider 16 is moved to push the projection, and which then turns on the power to the analyzing device 1. When the slider 16 is in the initial position, the actuator 22 is not operated.

A pair of stepped guide portions 23 are formed at the opening 11 on opposite sides of the bottom surface 111 parallel to the sensor pack 2 insertion direction. When the sensor pack 2 is inserted, the guide portions 23 guide the sensor pack 2 and restrain sideways the indented portion 61 of the base 6 to position the sensor pack 2 and the sensor chip 3 enclosed in the sensor pack 2 in a direction perpendicular to the direction of insertion.

The sensor chip 3 having the reagent layer 34 capable of reacting with a test sample is enclosed in a protected state in the packaging material 4. The sensor chip 3 is inserted into the analyzing device 1 in the state of being set in the sensor pack 2 before being taken out from the packaging material 4, the sensor pack 2 being inserted through the opening 11 of the analyzing device 1 in the direction of arrow A along the guide portions 23. The inserted sensor chip 3 is retained by the retaining member 13 in the analyzing device 1. The operator holds the packaging material 4 in this state between his or her fingers and pulls it in the direction of arrow B to remove only the packaging material 4 out of the analyzing device 1. The sensor chip 3 is left in the analyzing device 1 in the retained state. Thus, the operator can complete setting of the sensor chip 3 in the analyzing device 1 only by inserting and drawing out the sensor pack 2 without directly taking out the sensor chip 3.

A display portion 10 for displaying information on a result of analysis, or the like, is formed in an upper surface la of the analyzing device 1. A portion of the opening 11 is formed in the upper surface la to enable the sensor pack 2 insertion position to be easily recognized from the upper surface la side even when the analyzing device 1 is placed so that the display portion 10 is closer to the operator.

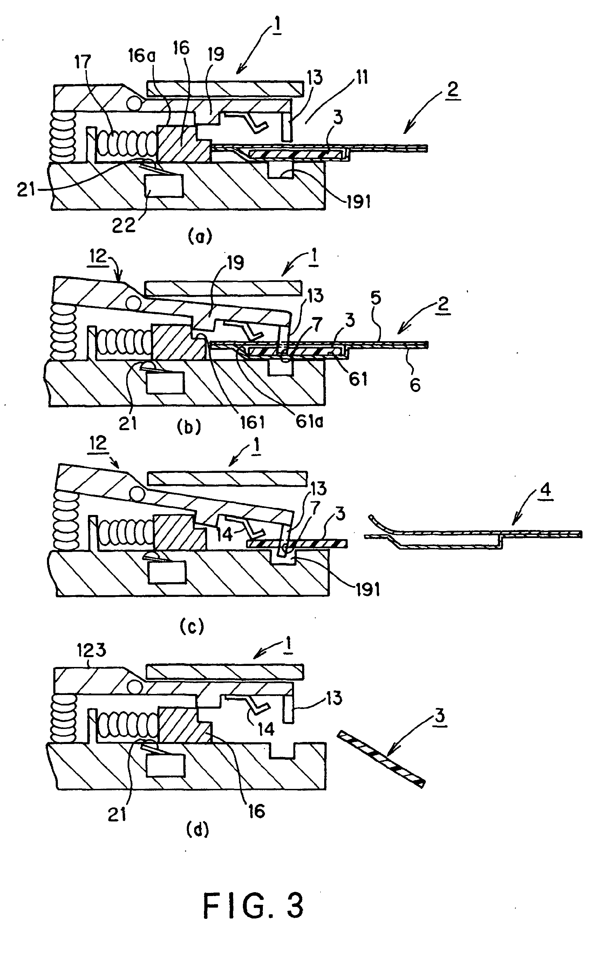

Figs. 3(a) to 3(d) are diagrams showing the operations of the portions of the analyzing device 1 when the sensor pack 2 is inserted into the analyzing device 1.

First, as shown in Fig. 3(a), the sensor pack 2 is inserted through the opening 11 of the analyzing device 1 to be brought its inserted end into contact with the slider 16.

At this time, the slider 16 is in the initial position, the lower surface 19a of the projection 19 and the upper surface 16a of the slider 16 are in contact with each other, and the retaining member 13 is not in contact with the sensor pack 2. Also, the actuator 21 of the switch 22 is not operated.

Next, as shown in Fig. 3(b), the sensor pack 2 in contact with the slider 16 is further moved to an inner portion of the analyzing device 1 in the insertion direction against the spring 17. The projection 19 of the supporting member 12 then falls from the upper surface 16a to the stepped portion 161 of the slider 16, so that the supporting member 12 rotates clockwise. The retaining member 13 is thus caused to move downward to tear the film (penetrable portion) 5. Simultaneously, the actuator 21 is operated to turn on the power. The sensor chip 3 has a hole 7 formed so as to be fitted around the retaining member 13. The retaining member 13 moving downwardly plunges through the hole (engagement means) 7 of the sensor chip 3. The retaining member 13 plunges through the hole 7 of the sensor chip 3 to increase the retaining force, thereby reliably retaining the sensor chip 3. Since the base (penetration limiting portion) 6 is a hard member, the retaining member 13 is stopped when brought into contact with the base 6 (first state). Since the height of the connection electrodes 14 from the lower surface 121a of the supporting member 12 is smaller than that of the retaining member 13 by such an amount that the connection electrodes 14 do not contact or contact the sensor pack 2 only loosely, and do not contact the sensor chip 3. Thus, some fat or oil or other contaminants attached to the film 5 are prevented from being attached to the connection electrodes. Also, the resistance to the action of drawing out the packaging material 4 may be limited. In this embodiment, the supporting member 12, the spring 20, the slider 16 and the spring 17 constitute means for changing the state of the retaining means.

When the sensor pack 2 is drawn out of the opening 11 of the analyzing device 1 after the sensor chip 3 has been set in the state of being retained by the retaining member 13, the retaining member 13 continues retaining the sensor chip 3 while tearing the film 5. As the packaging material 4 is drawn out, the sensor chip 3.moves upward along a sloping surface 61a of the indented portion 61 on the analyzing device insertion side of the base 6. When the sensor chip 3 comes to the surface of the base 6 bonded to the film 5, it tears the film 5 by its edge to get out of the packaging material 4. At this time, if the connection electrodes 14 are in close contact with the sensor pack 2, the resistance to the drawing action is considerably large. It is, therefore, desirable that the contact electrodes 14 do not contact or contact the sensor pack 2 only loosely, as mentioned above.

A recess 191 is formed in the bottom surface 19 of the analyzing device 1 at a position in the vicinity of the opening 11 corresponding to the retaining member 13. When the packaging material 4 is removed from the analyzing device 1, the supporting member 12, which has been stopped by the base 6 from rotating, further rotates clockwise, so that, as shown in Fig. 3(c), the retaining member 13 enters the recess 191 of the analyzing device 1 and the connection electrodes 14 are brought into contact with the sensor chip 3 (second state). The positional relationship between the retaining member 13 and the connection electrodes 14 in the supporting member 12 at this time is set so as to correspond to the positional relationship between the hole 7 and the terminal electrode portions 32c in the sensor chip 3. Therefore, when the retaining member 13 falls in the recess 191, the connection electrodes 14 and the terminal electrode portions 32c automatically contact each other to establish electrical connections therebetween. In this embodiment, the retaining member 13, the base 6 and the recess 191 constitute means for changing the state of the connection electrodes (reaction information acquisition means).

Thus, in this state, the reagent layer 34 and the reaction electrode portions 32a,32a of the sensor chip 3 are exposed, the power is turned on, and the reaction electrode portions 32a are electrically connected, thereby enabling a measurement to be immediately carried out.

When the sensor chip 3 is thrown away after the completion of the measurement, a button (retention undoing means) 123 provided in the upper surface of the arm 122 of the supporting member 12 opposite from the opening 11 is pressed. The supporting member 12 is thus rotated counterclockwise against the action of the spring 20, as shown in Fig. 3(d), thereby disengaging the retaining member 13 and the connection electrodes 14 from the sensor chip 3. In this state, the sensor chip 3 can be immediately made to fall into a waste container. Thus, the sensor chip 3 can be thrown away with no need for touching the sensor chip 3. When the supporting member 12 rotates counterclockwise after pressing of the button 123, the projection 19 also moves upward to recede from the stepped portion 161. Then the slider 16 is pressed and moved toward the opening 11 by the urging force of the spring 17 to return to the initial position. Also, the actuator 21 returns to the initial position, and the power is turned off.

### (Second Embodiment)

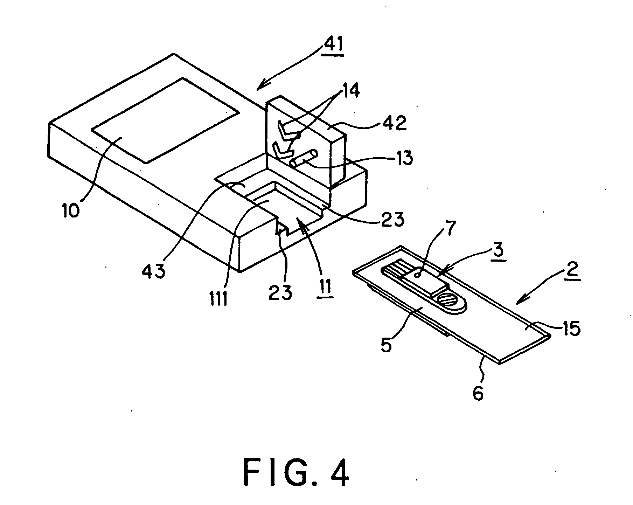

Fig. 4 shows an analyzing device 41 and a sensor pack 2 in a second embodiment of the present invention.

The same components or portions as those in the first embodiment are indicated by the same reference characters, and the description for them will be omitted.

The constructions of the sensor pack 2 and the sensor chip 3 of this embodiment are the same as those in the first embodiment.

The configurations of the retaining member 13 and the connection electrodes 14 of the analyzing device 41 are the same as those in the first embodiment. In this embodiment, however, a supporting member 42 is capable of opening and closing by moving relative to the main body of the analyzing device 41.

The supporting member 42 is connected to the main body of the analyzing device 41 by a hinge or the like, thereby being made swingable along a plane perpendicular to the sensor pack 2 insertion direction. The pair of guide portions 23,23 are formed at the opening 11 on opposite sides of the bottom surface, as are those in the first embodiment. An inner stepped portion is also formed continuously with the guide portions 23,23 thereby forming an end stop portion 43 for stopping the end of the indented portion 61 of the sensor pack 2 in the insertion direction.

The method of setting the sensor chip 3 in this embodiment will now be described.

First, as shown in Fig. 4, the sensor pack 2 is placed on the opening bottom surface 111, with the supporting member 42 opened. At this time, the guide portions 23,23 and the end stop portion 43 restrain the insertion-direction end and the two side portions of the indented portion 61, thereby positioning the sensor pack 2.

Next, the supporting member 42 is closed to make the retaining member 13 retain the sensor chip 3 by tearing the film 5 and by plunging through the hole 7 of the sensor chip 3. Thereafter, the holdable extension 15 of the sensor pack 2 is held and pulled to take out the sensor chip 3 while tearing the film 5. The terminal electrode portions 32c,32c of the sensor chip 3 taken out contact the connection electrodes 14,14 to establish electrical connection therebetween, thereby enabling a measurement to be carried out by supplying a test sample to the reagent layer 34, for example, by dropping the sample.

After the completion of the measurement, the supporting member 42 is opened to disengage the sensor chip 3 from the retaining member 13, thereby enabling the sensor chip 3 to be thrown away. Since the entire sensor chip 3 is exposed when the supporting member 42 is opened, it is possible to throw away the sensor chip 3 by holding a suitable portion of the sensor chip 3. In this manner, the sensor chip 3 can be thrown away without a risk of touching a test sample in a case where the test sample is a blood sample.

While in this embodiment the supporting member 42 is opened and closed along a plane perpendicular to the sensor pack 2 insertion direction, the supporting member 42 may alternatively be opened and closed along a plane parallel to the sensor pack 2 insertion direction.

### (Third Embodiment)

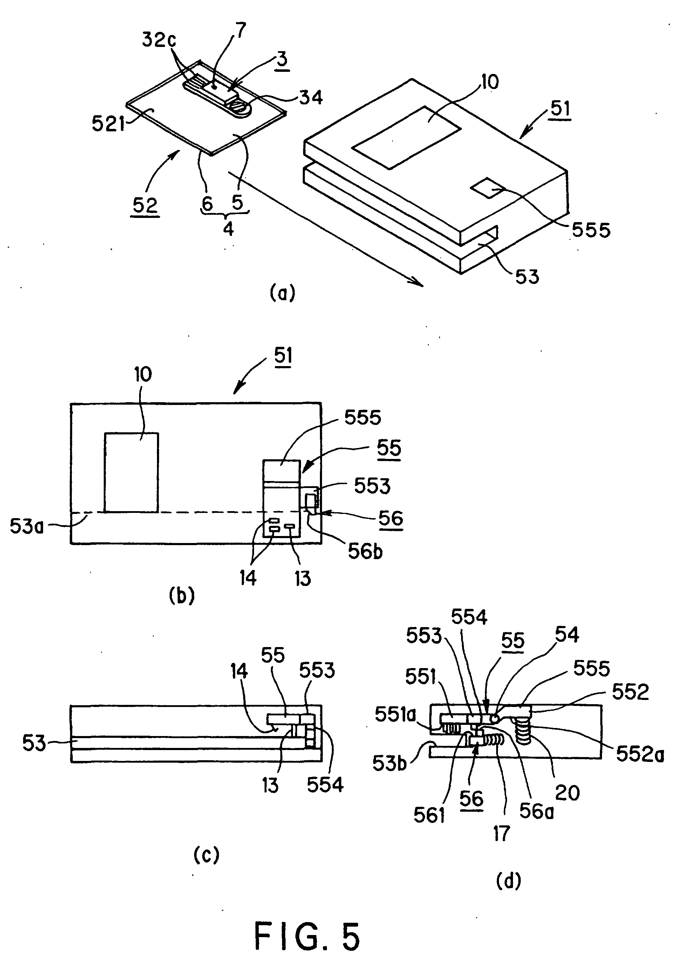

Figs. 5(a) through 5(d) show an analyzing device 51 and a sensor pack 52 in a third embodiment of the present invention.

The same components or portions as those in the first embodiment are indicated by the same reference characters, and the description for them will be omitted.

In this embodiment, a groove 53 is formed in a side portion of the analyzing device 51, and the sensor pack 52 is inserted into the groove 53 in the direction of the arrow and is passed through the groove 53 to set the sensor chip 3.

Fig. 5(a) shows the entire configuration of the analyzing device 51 and the sensor pack 52. Fig. 5(b) is a top view of the analyzing device 51, Fig. 5(c) is a side view as seen from the bottom side of Fig. 5(b), and Fig. 5(d) is a side view as seen from the right-hand side of Fig. 5(b). These figures schematically show only the construction of essential portions and the external configuration of the analyzing device 51 without showing other constituent portions.

A supporting member 55 is provided above an end portion of the groove 53 in the back side of the insertion entrance swingably supported around a shaft 54. A retaining member 13 and connection electrodes 14,14 are provided on a lower surface of a groove side arm portion 551 of the supporting member 55. A lower surface of an arm portion 552 of the supporting member 55 in an inner portion in the analyzing device 51 receives pressure from a spring 20. The retaining member 13 and the connection electrodes 14,14 are placed along the lengthwise direction of the groove 53. A slider 56 projecting from a side surface 53a of the groove 53 can move on a bottom surface 53b in a direction perpendicular to the lengthwise direction of the groove 53. When the slider 56 is pressed and displaced from an initial position shown in Figs. 5(b) and (d) to an inside of the analyzing device, it is pressed in the direction toward the groove 53 by a spring 17 provided inside of the device. A side surface of the projecting portion of the slider 56 exposed in the groove 53 is formed as a slanting surface 56b inclined in the sensor pack insertion direction projecting gradually into the groove, such that the sensor pack 52 inserted along the groove 53 can smoothly displace the slider 56 to the inside of the analyzing device. The groove side arm portion 551 of the supporting member 55 has an extension 553 located above the slider 56, and a projection 554 having a generally rectangular cross section is formed on a lower surface of the extension 553. A stepped portion 561 capable of fittingly receiving the projection 554 is formed in the upper surface of the slider 56 on the groove 53 side.

The construction of the sensor pack 52 is substantially the same as that in the first embodiment. However, a holdable extension 521 to be held between operator's fingers is formed so as to extend in a direction along the width direction of the sensor chip 3 (in a direction perpendicular to the insertion direction) to enable an operation for inserting the sensor pack 52 to the groove 53 and moving the sensor pack 52 within the groove 53 so that the lengthwise direction of the sensor chip 3 is parallel to the groove 53. Since in this embodiment the sensor pack 52 is inserted in and passed through the groove 53, the end of the indented portion (not shown) of the base 6 of the packaging material 4 opposite from the end in the insertion direction is formed to have a slanting surface.

The method of setting the sensor chip 3 in this embodiment will now be described.

First, the operator inserts the sensor chip 3 accommodation portion into the groove 53 of the analyzing device 51 by holding the holdable extension 521 of the sensor pack 52, and moves the sensor pack 52 along the side surfaces of the groove 53 in the direction of the arrow while maintaining the sensor pack 52 so that the lengthwise direction of the sensor chip 3 is parallel to the groove 53.

After the end of the sensor pack 3 facing the insertion direction has been brought into contact with the slider 56, the sensor pack 3 is further moved to displace the slider 56 to the inner side of the analyzing device. At this time, the projection 554 that has been maintained in contact with the slider upper surface 56a falls to the stepped portion 561 and the supporting member 55 rotates so that the groove side arm portion 551 moves downward into the groove 53. The retaining member 13 is thereby caused to plunge through the hole 7 of the sensor chip 3 to retain the sensor chip 3.

When the sensor pack 52 is further moved, the film 5 is torn by the sensor chip 3 retained, and only the packaging material 4 is removed, with the retained sensor chip 3 left in the analyzing device. The connection electrodes 14,14 and the terminal electrode portions 32c, 32c contact each other to establish electrical connections therebetween, and the reagent layer 34 and the reaction electrode portions 32a are exposed outside a side surface. Thereafter, a measurement can be carried out by supplying a test sample in a dropping manner, or the like.

When the button 555 is depressed after the completion of the measurement, the supporting member 55 rotates against the action of the spring 20, so that the retaining member 13 and the connection electrodes 14,14 recede from inside of the groove 53. Also, the projection 554 recedes from the stepped portion 561, thereby allowing the slider 56 to move toward the inside of the groove 53 to return to the initial position. After release of the button 555, the projection 554 is again brought into contact with the upper surface 56a of the slider 56 to be restrained from rotating. With receding of the retaining member 13 from inside of the groove 53, the sensor chip 3 is disengaged from the retaining member 13, thus enabling the operator to throw away the sensor chip 3 without touching the sample.

While in this embodiment the groove 53 is formed in a side surface of the.analyzing device 51, the groove 53 may alternatively be formed in the upper surface in which the display portion 10 is provided, or in some other surface. Also in the case of forming the groove 53 in the upper surface, the same internal mechanisms, including the mechanism for retaining the sensor chip 3, can be used. The method of passing the sensor pack 52 through the groove 53 in one direction is not exclusively used. A method of inserting the sensor pack 52 from one direction and drawing out the sensor pack 52 in the opposite direction may alternatively be used.

### (Fourth Embodiment)

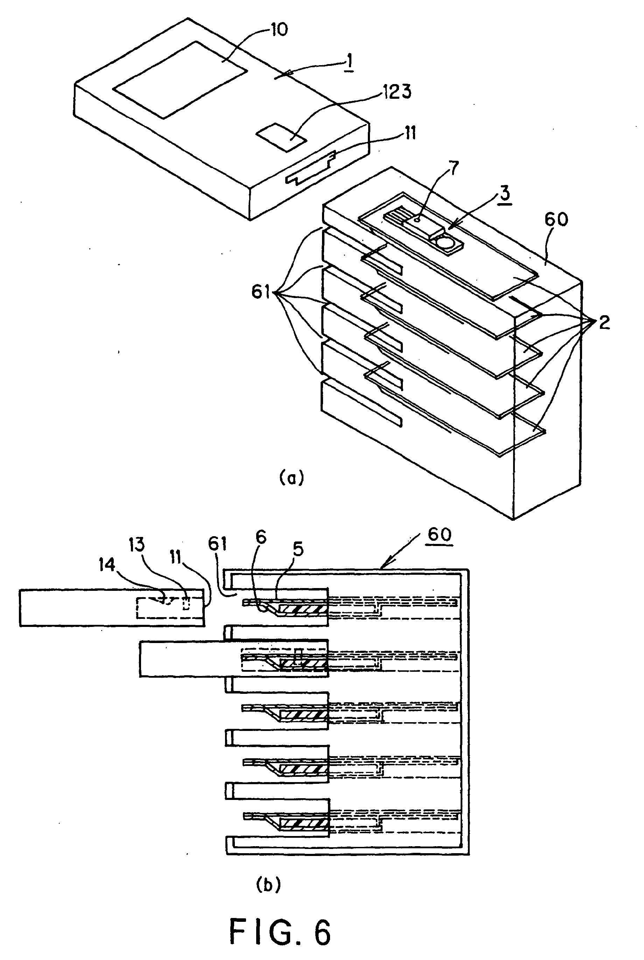

Figs. 6(a) and (b) show an analyzing device 1 and sensor packs 2 in a fourth embodiment of the present invention.

The same components or portions as those in the first embodiment are indicated by the same reference characters, and the description for them will be omitted.

In this embodiment, as shown in Fig. 6(a), a plurality of sensor packs 2 are accommodated in one case 60. In the case 60, a slit 61 in which the analyzing device 1 is inserted is formed in correspondence with each sensor pack 2. Each sensor pack 2 has its portion on the insertion side in the slit 61 and has the other end portion retained in the case.

The analyzing device 1 in this embodiment is the same as that in the first embodiment except that the opening 11 is formed so as to open in only one side surface.

The method of setting the sensor chip 3 in this embodiment will be described.

As shown in Fig. 6(b), the analyzing device 1 is inserted into one of the slits 61 of the case 60, with its opening 11 facing the slit. At this time, the insertion side end of the sensor pack 2 exposed to the slit 61 is inserted into the opening 11. When the analyzing device 1 is inserted to bring its end into contact with a side surface of the groove 61, the sensor pack is also inserted to a predetermined position in the opening 11. At this time, the insertion side end of the sensor pack 2 pushes in the slider (not shown), so that the supporting member (not shown) rotates, and the retaining member 13 plunges through the hole 7 of the sensor chip 3 to retain the sensor chip 3.

Next, the analyzing device 1 is drawn out from the slit 61. The sensor chip 3 is thereby taken out from the packaging material by tearing the film 5 and is fully extracted while being retained in the analyzing device 1. Since the film 5 and the base 6 are retained in the case 60, they are left together with the case 60. In this state, the connection electrodes 14 and the terminal electrode portions 32c contact each other to establish electrical connections therebetween, so that a measurement can be carried out by supplying a test sample to the reagent layer 34 exposed outside the opening 11, such as by dropping the sample onto the reagent layer 34.

After the completion of the measurement, the button 123 is depressed to disengage the retaining means 13 from the hole 7 of the sensor chip 3, as in the first embodiment, thereby enabling the sensor chip 3 to be thrown away by being put into a waste container or the like with no need for touching the sensor chip 3.

If a plurality of sensor chips 3 are accommodated in one case 60 as described above, they can be collectively accommodated, managed, and the possibility of losing a sensor pack is reduced.

### (Fifth Embodiment)

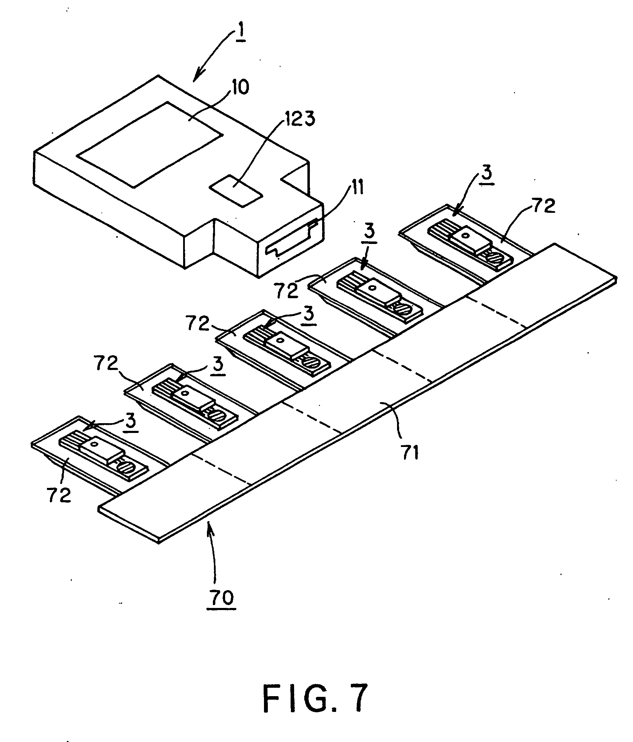

Fig. 7 shows an analyzing device 1 and a sensor pack 70 in a fifth embodiment of the present invention.

The same components or portions as those in the first embodiment are indicated by the same reference characters, and the description for them will be omitted.

The sensor pack 70 in this embodiment has the same sensor chip accommodation structure as that in the first embodiment. In the sensor pack 70, the ends of a plurality of sensor chip accommodation portions 72 opposite the insertion side are connected by a connecting portion 71, and a sensor chip 3 is accommodated in each of the accommodation portions 72 extending like comb teeth from the connecting portion 71. The analyzing device 1 also has generally the same construction as that in the first embodiment. However, the analyzing device 1 in this embodiment differs from that in the first embodiment in that the side surface in which the opening 11 is formed is cut at its ends opposite from each other in the horizontal direction so as to avoid interference with the adjacent accommodation portion 72 and that the opening 11 is provided on only one side surface.

The method of setting the sensor chip 3 in this embodiment is the same as that in the first embodiment, and the description for it will be omitted.

If a plurality of sensor chips 3 are accommodated in one sensor pack 70 as described above, they can be collectively accommodated, managed, and the possibility of losing the sensor pack is reduced.

Perforations or the like may be provided in the connecting portion 71 to enable each accommodation portion to be easily separated from the others.

### (Sixth Embodiment)

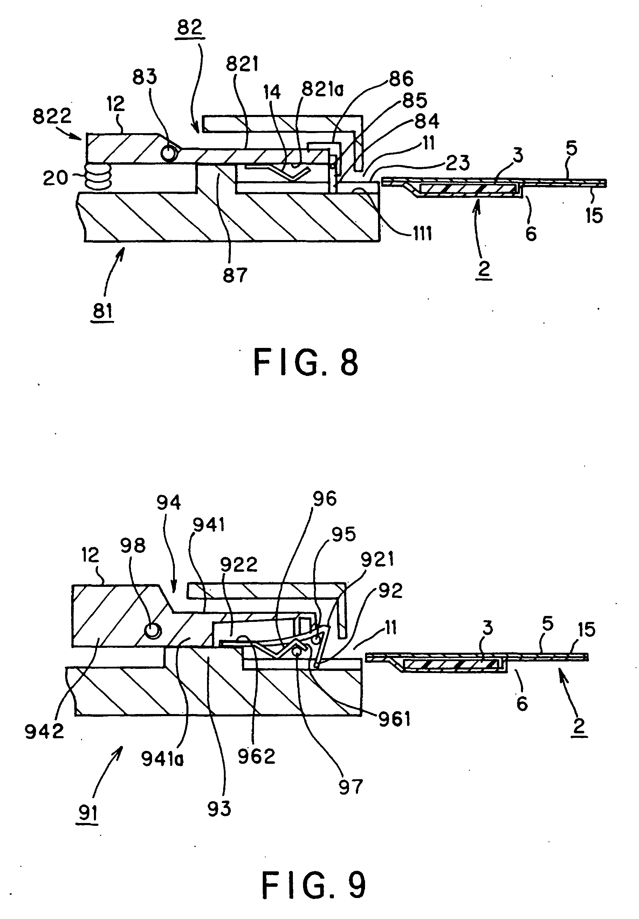

Fig. 8 is a cross-sectional view of an analyzing device and a sensor pack in a sixth embodiment of the present invention, schematically showing the construction of essential portions of the analyzing device and the construction of the sensor pack.

The construction of the sensor pack 2 is the same as that in the first embodiment. The analyzing device 81 is the same as the analyzing device 1 in the first embodiment except for the construction of a retaining member 84 and a supporting member. The same components or portions as those in the first embodiment are indicated by the same reference characters, and the description for them will be omitted.

The supporting member 82 is provided swingably around a shaft 83. The retaining member (retaining means) 84 is provided on a tip end of an opening 11 side arm portion 821 of the supporting member 82 so as to be swingable around a shaft 85 to the insertion direction. The counterclockwise rotation of the retaining member 84 from the initial position shown in Fig. 8 is stopped by the contact of an upper end portion of the retaining member 84 with an end surface of the supporting member 82. Therefore, the retaining member 84 can rotate only clockwise from the initial position. A lower end portion of the retaining member 84 and the opening side arm portion 821 are connected by a spring 86. The spring 86 urges the retaining member 84 counterclockwise when the retaining member 84 rotates clockwise from the initial position. Connection electrodes 14 are provided on a lower surface 821a of the arm portion 821 of the supporting member 82 at an inner side of the analyzing device relative to the retaining member 84. A portion of the arm portion lower surface 821a inside the analyzing device relative to the connection electrodes 14 is in contact with a projection 87 projecting from an opening bottom surface 111 and having a generally rectangular cross section, thereby stopping the clockwise rotation of the supporting member 82 from the initial position shown in Fig. 8. An externally exposing button 12 is formed on the upper surface of an inner arm portion 822 of the supporting member 82. A lower surface of the inner side arm portion 822 receives pressure from a spring 20 interposed between the lower surface and the bottom surface, thereby urging the supporting member 82 to rotate clockwise.

The method of setting the sensor chip 3 in this embodiment will now be described.

First, the sensor pack 2 is inserted through the opening 11 along the guide portions 23. At this time, the retaining member 84 swings clockwise by being pressed by the sensor pack 2, so that the retaining member 84 does not impede the insertion of the sensor pack 2.

After the sensor pack 2 has been inserted to be brought into contact with the projection 87, the button 12 is pressed to rotate counter-clockwise the opening side arm portion 821 of the supporting member 82. At this time, the retaining member 84 is rotated counterclockwise by the spring 86 to be returned to the initial position. When the button 12 is released, the supporting member 82 rotates clockwise by the action of the spring 20, so that the retaining member 84 tears the film 5 and plunges through the hole (not shown) of the sensor chip 3 to retain the sensor chip 3.

Next, the sensor pack 2 is drawn out by holding the holdable extension 15. The sensor chip 3 is thereby taken out from the packaging material 4 while tearing the film 5 to remain in the analyzing device 81 in the retained state. Only the packaging material 4 is thereby removed. Since counterclockwise swinging of the retaining member 84 from the initial position is stopped, it is not possible that the retaining member 84 will be disengaged from the hole of the sensor chip 3 when the packaging material 4 is drawn out. The connection electrodes 14 and the terminal electrode portions (not shown) contact each other to establish electrical connections therebetween, thereby enabling a measurement to be carried out by supplying a sample to the reagent layer (not shown) exposed outside the opening 11, such as by dropping the sample onto the reagent layer.

After the completion of the measurement, the button 12 is pressed to disengage the retaining member 84 from the hole of the sensor chip 3, thereby enabling the sensor chip 3 to be thrown away by being put into a waste container or the like with no need to directly touch the sensor chip 3.

### (Seventh Embodiment)

Fig. 9 is a cross-sectional view of an analyzing device 91 and a sensor pack 2 in a seventh embodiment of the present invention, schematically showing the construction of essential portions of the analyzing device and the construction of the sensor pack.

The construction of the sensor pack 2 is the same as that in the first embodiment. The analyzing device 91 is the same as the analyzing device 1 in the first embodiment except for the constructions of a retaining member, connection electrodes and a supporting member. The same components or portions as those in the first embodiment are indicated by the same reference characters, and the description for them will be omitted.

The retaining member (retaining means) 92 is formed of a flexible member in the form of an angled rod. The retaining member 92 is substantially v-shaped having a bent portion 921 and a fixed portion 922. The fixed portion 922 is fixed on a projection 93 projecting from a bottom surface 111 of an opening 11 and having a generally rectangular cross section. A lifting lever 95 of the supporting member 94 is engaged with the inner surface of the bent portion 921. The lifting lever 95 is extended from an opening side arm portion 941 of the supporting member 94 in a direction perpendicular to the insertion direction (in a direction perpendicular to the plane of projection of the figure). In the state shown in Fig. 9, the retaining member 92 is bent counterclockwise on the fixed portion 922 side.

Each of the connection electrodes (reaction information acquisition means) 96 is formed of a flexible plate member generally V-shaped by being bent upwardly. Each connection electrode 96 has a bent portion 961 downwardly bent on the opening 11 side, and a fixed portion 962 extending toward a center of the analyzing device. A lifting lever 97 of the supporting member 94 extended from the opening side arm portion 941 of the supporting member 94 in a direction perpendicular to the insertion direction is engaged with the inner surface of the opening 11 side bent portion 961. The fixed portion 962 of each connection electrode 96 is fixed on the projection 93. (The structure of electrical connections of the connection electrodes to inner portions of the analyzing device are not illustrated.)

The supporting member 94 is swingably provided about a shaft 98. The supporting member 94 has the above-mentioned lifting levers 95 and 97 on its opening side arm portion 941, and a contact portion 941a which is an opening side lower surface portion in the vicinity of the shaft 98, and which contacts the upper surface of the projection 93 to stop the clockwise rotation of the supporting member 94. An externally-facing button 12 is formed on the upper surface of an inner side arm portion 942 of the supporting member 94. In the state shown in Fig. 9, the supporting member 94 is urged clockwise by bending of the retaining member 92 and the connection electrodes 96.

The method of setting the sensor chip 3 in this embodiment will now be described.

First, as shown in Fig. 9, the sensor pack 2 is inserted through the opening 11 while the contact portion 941a of the supporting member 94 contacts the projection 93 to stop the rotation of the supporting member 94. Since the retaining member 92 has flexibility, it is bent clockwise from the bent portion 921 by being pressed by the sensor pack 2. The connection electrodes 96 are bent counterclockwise from the fixed portions 922 by being pressed by the sensor pack 2. Therefore, the insertion of the sensor pack 2 is not impeded.

Next, when the button 12 is pressed, the supporting member 94 rotates counterclockwise to lift the retaining member 92 and the connection electrodes 96 by the lifting levers 95 and 97. At this time, the retaining member 92, which has been bent, is restored to the initial straight state. Thereafter, when the button 12 is released, the supporting member 94 rotates clockwise by the urging force of the retaining member 92 and the connection electrodes 96, and the retaining member 92 tears the film 5 and plunges through the hole (not shown) of the sensor chip 3 to retain the sensor chip 3.

When the sensor pack 2 is drawn out by holding the holdable extension 15, the sensor chip 3 is taken out from the packaging material 4 while tearing the film 5 to remain in the analyzing device 91. Only the packaging material 4 is thereby removed. At this time, the connection electrodes 96 and the terminal electrode portions (not shown) of the sensor chip 3 contact each other to establish electrical connections therebetween, and the reagent layer (not shown) is exposed outside the opening 11, thereby enabling a measurement to be carried out by supplying a sample to the reagent layer, such as by dropping the sample onto the reagent layer.

After the completion of the measurement, the button 12 is pressed to disengage the retaining member 92 from the hole of the sensor chip 3, thereby enabling the sensor chip 3 to be thrown away by being put into a waste container or the like with no need for directly touching the sensor chip 3.

In the above-described embodiments, the retaining member is engaged with the sensor chip by being made to plunge through the hole of the sensor chip. Alternatively, a recess or projection may be provided on the sensor chip and the retaining member may be made to engage with the recess or the projection.

The cross-sectional shape of the retaining member may be selected appropriately. However, for the purpose of tearing the film at the time of drawing out the sensor pack, it is desirable that the retaining member should have a shape suitable for tearing the film, such as a circular or elliptical cross section, a rectangular cross section whose lengthwise direction corresponds to the direction of drawing out, or a wedge-shaped cross section.

### (Eighth Embodiment)

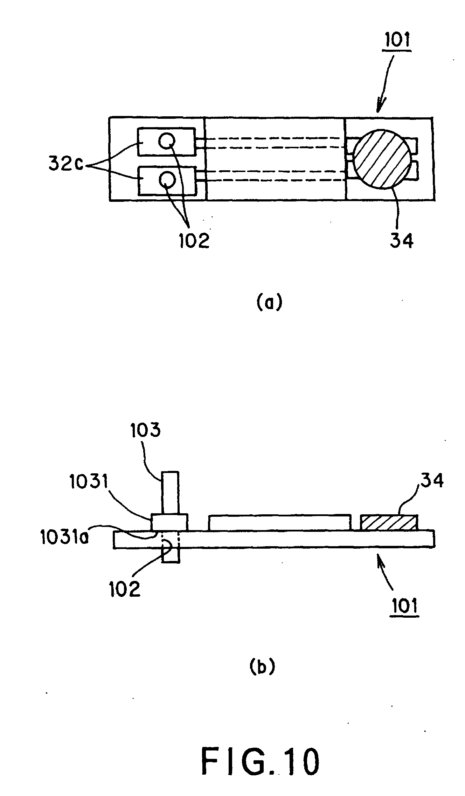

Figs. 10(a) and 10(b) show a sensor chip and a retaining member in an eighth embodiment of the present invention.

The construction of essential portions of the analyzing device is substantially the same as that in the first embodiment except for the construction of the retaining member and the connection electrodes. The same components or portions as those in the first embodiment are indicated by the same reference characters, and the description for them will be omitted.

The sensor chip 101 of this embodiment have two holes (engagement means) 102,102 respectively formed in the pair of terminal electrode portions 32c,32c to be engaged with retaining members 103, as shown in Fig. 10(a). Except for the arrangement of the holes 102, the other constructions of the sensor chip 101 is the same as that in the first embodiment.

Fig. 10(b) shows a state in which retaining members (retaining means) 103 are fitted in the holes 102. Two retaining members 103 are arranged in the width direction of the sensor chip 101 (in a direction perpendicular to the insertion direction) in correspondence with the holes 102. Each retaining member 103 is formed of a conductive material, has the shape of a rod, and has a large-diameter portion 1031. An end surface of the large-diameter portion 1031 is brought into contact with the terminal electrode portion 32c to function as a connection electrode. If a retaining member and a connection electrode are formed integrally with each other as described above, the construction of the device can be simplified and the number of component parts can be reduced.

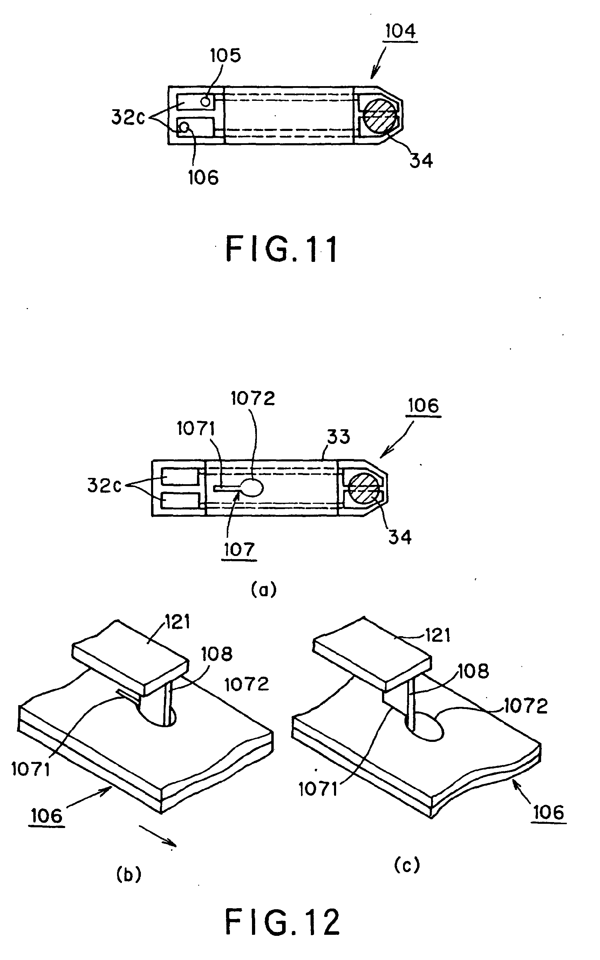

Fig. 11 shows a sensor chip 104 which is an example of a modification of this embodiment.

The sensor chip 104 has holes (engagement means) 105 and 106 respectively formed in terminal electrode portions 32c,32c to be engaged with the retaining members. The positions of the two holes 105 and 106 are shifted relative to each other along the lengthwise direction (insertion direction) of the sensor chip 104. Except for the holes 105 and 106, the construction of the sensor chip 104 is the same as that of the sensor chip 101. Correspondingly, the retaining members each having the same construction as the above-described retaining member 103 are positioned by being shifted from each other along the insertion direction of the sensor chip 104. If the holes to be engaged with the retaining members are placed so as to be asymmetrical about a line as described above, the sensor chip is not retained when inadvertently inserted in the reversed state, thus preventing insertion in the reversed state.

### (Ninth Embodiment)

Figs. 12(a), 12(b), and 12(c) show a sensor chip 106 and a retaining member 108 in a ninth embodiment of the present invention.

The same components or portions as those in the first embodiment are indicated by the same reference characters, and the description for them will be omitted. The construction of essential portions of the analyzing device is substantially the same as those in the first embodiment except for the construction of the retaining member and the connection electrodes.

Fig. 12(a) shows the sensor chip 106 of this embodiment. The construction of the sensor chip 106 is the same as that in the first embodiment except for the shape of a hole 107 (engagement means) to be engaged with the retaining member. The hole 107 has a rectangular portion 1071 formed on the insertion side (on the terminal electrode portion 32c side) and having a generally rectangular shape with a lengthwise direction corresponding to the insertion direction, and a circular portion 1072 formed on the opposite side in the insertion direction (on the reaction portion 8 side) having a generally circular shape.

Figs. 12(b) and 12(c) show states of fitting of the hole 107 and the retaining member (retaining means) 108. In this embodiment, the retaining member 108 has the shape of a plate extending perpendicularly to an opening-side arm portion 121 of the supporting member and having a cross section elongated along the insertion direction. When a sensor pack (not shown) is inserted and the arm portion 121 of the supporting member rotates to fit the retaining member 108 in the hole 107, the retaining member 108 is fitted in the circular portion 1072, as shown in Fig. 12(b). Next, when the sensor pack is drawn out, the sensor chip 106 is also drawn in the direction of the arrow opposite to the insertion direction, so that the retaining member 108 moves from the circular portion 1072 to the rectangular portion 1071. At the time of insertion of the sensor pack, the retaining member 108 is engaged with the circular portion 1072 and this engagement can be reliably effected even if a certain error occurs in positioning of the retaining member 108. At the time of setting of the sensor chip 106, the retaining member 108 is engaged with the rectangular portion 1071 to be able to retain the sensor chip 106 without play in the direction perpendicular to the insertion direction.

### (Tenth Embodiment)

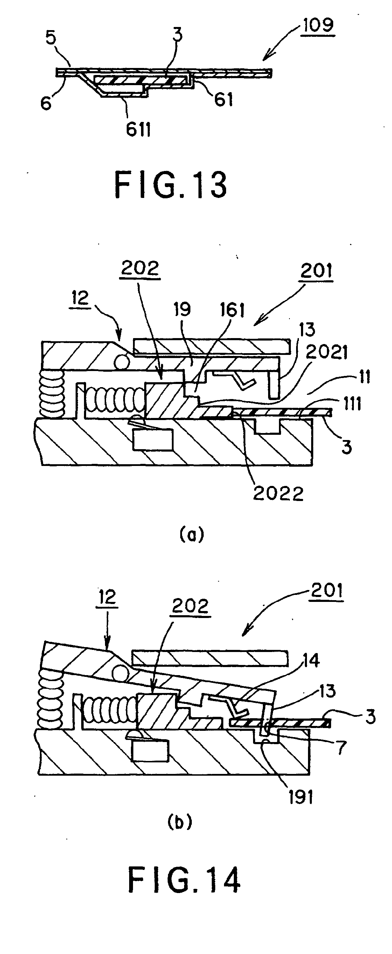

Fig. 13 shows a sensor pack 109 configuration in a tenth embodiment of the present invention. The description for the same components or portions as those in the first embodiment will be omitted. The constructions of the sensor chip and the analyzing device are the same as those in the first embodiment.

In the sensor pack 109, an additional indented portion 611 one step lower than the indented portion 61 is formed at a position below the hole (not shown) of the sensor chip 3 and on the adjacent portion of this in the insertion direction. If such a indented portion 611 is formed, the retaining member (not shown) plunging through the hole of the sensor chip 3 is inserted more deeply and engaged with the hole more reliably, thereby enabling securer retention of the sensor before the sensor pack 109 is taken out from the packaging material.

### (Eleventh Embodiment)

Figs. 14(a) and 14(b) show the construction of essential portions of an analyzing device 201 in an eleventh embodiment of the present invention. The same components or portions as those in the first embodiment are indicated by the same reference characters, and the description for them will be omitted. The construction of the sensor pack and the sensor chip is the same as that in the first embodiment.

The analyzing device 201 is the same as that in the first embodiment except for the construction of a slider 202. The slider 202 has, on the opening 11 side, an upper stepped portion 161 capable of fittingly receiving the projection 19 of the supporting member 12, a lower sensor pack contact surface 2021 which is formed adjacent to the stepped portion 161, and against which the sensor pack (not shown) is brought into contact, and a sensor chip contact portion (positioning means) 2022 which is formed adjacent to the sensor pack contact surface 2021, and which projects from a lower portion of the slider toward the opening 11 side along the opening bottom surface 111.

Since the indented portion 61 for accommodating the sensor chip 3 is provided in the sensor pack next to the edge of the base 6, the insertion-side end portion of the inserted sensor pack is at a height corresponding to the depth of the indented portion 61 from the bottom surface 111 of the opening 11 (see Fig. 2). Therefore, when the sensor pack is inserted through the opening 11 of the analyzing device 109, the insertion-side end of the sensor pack is brought into contact against the sensor pack contact surface 2021 of the slider 202. Even in a case where the process of opening the sensor pack, taking out the sensor chip 3 and inserting only the sensor chip 3 through the opening 11 of the analyzing device 201 is performed by mistake, the sensor chip 3 may be inserted along the bottom surface 111 to be brought into contact against the sensor chip contact portion 2022 and to push in the slider 202. Since in the sensor pack the sensor chip 3 is accommodated by being positioned in the indented portion, the amount of projection of the sensor chip contact portion 2022 is set in correspondence with the distance between the insertion-side end of the sensor pack and the insertion-side end of the sensor chip 3, thereby ensuring that the retaining member 13 can plunge through the hole 7 of the sensor chip 3 and then plunge into the recess 191, and the connection electrodes 14 can contact the terminal electrode portions (not shown) in the correct positional relationship with each other.

Thus, even in a case where an operator takes out the sensor chip 3 from the sensor pack by mistake, he or she may hold the sensor chip 3, insert the sensor chip 3 through the opening 11 to press the sensor chip contact portion 2022 of the slider 202, thereby enabling the sensor chip 3 to be reliably retained and used for a measurement. Thus, the possibility of wasting the sensor chip is reduced.

### (Twelfth Embodiment)

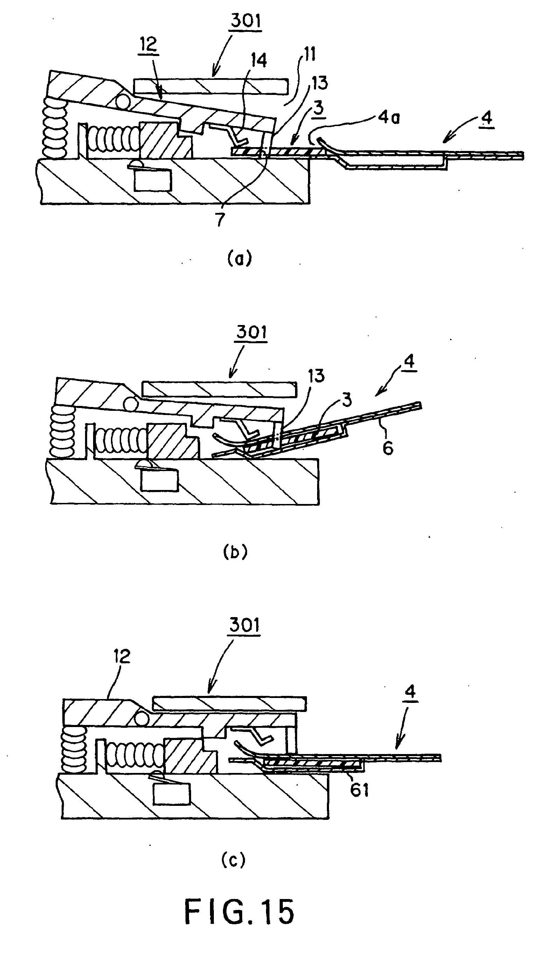

Figs. 15(a), 15(b), and 15(c) show the construction of essential portions of an analyzing device, a sensor chip 3, and a packaging material in a twelfth embodiment of the present invention.

The same components or portions as those in the first embodiment are indicated by the same reference characters, and the description for them will be omitted. The construction of the sensor chip and the packaging material is the same as that in the first embodiment.

The construction of essential portions of the analyzing device in this embodiment is the same as that in the first embodiment except the that recess in the opening bottom surface at the position corresponding to the retaining member is removed.

As shown in Fig. 15(a), after the sensor chip 3 has been set and a measurement using the sensor chip 3 has been completed, the sensor chip 3 is inserted into an extraction opening 4a of the packaging material 4, through which the sensor chip 3 has previously been taken out, while the sensor chip 3 is maintained in the state of being retained by the retaining member 13. At this time, since no recess is formed in the bottom surface 111 of the opening 11 of the analyzing device 301, the retaining member 13 plunging through the hole 7 of the sensor chip 3 is brought into contact against the bottom surface 111.

As the packaging material 4 is further inserted, as shown in Fig. 15(b), the sensor chip 3 enters the packaging material 4 and the retaining member 13 is lifted by being scooped by the base 6 of the packaging material 4,.

After the sensor chip 3 has been accommodated in the indented portion 61 of the packaging material 4, the button 123 is pressed to disengage the retaining member 13 from the sensor chip 3, thereby enabling the sensor chip 3 to be taken out in the state of being accommodated in the packaging material 4.

### (Thirteenth Embodiment)

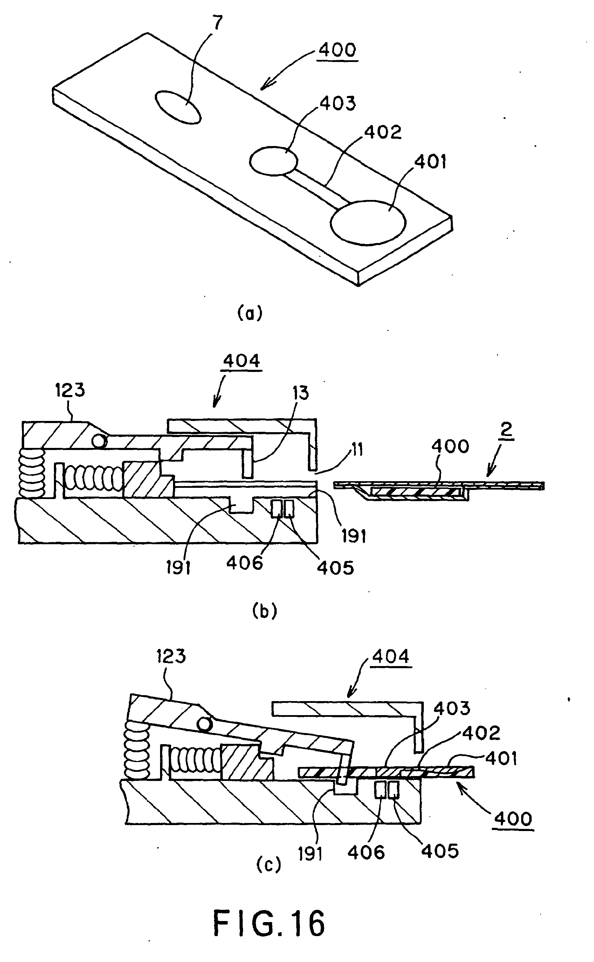

Figs. 16(a), 16(b), and 16(c) show a sensor chip, a sensor pack and an analyzing device in a thirteenth embodiment of the present invention. The same components or portions as those in the first embodiment are indicated by the same reference characters, and the description for them will be omitted.

In the above-described embodiments, the sensor chip is of such a type that an electrochemical phenomenon resulting from a reaction between a reagent in the reaction portion and a particular ingredient in a test sample is detected through the electrodes at the reaction portion. In this embodiment, the present invention is applied to an optical reading type of analyzing device and a sensor chip for use in the analyzing device.

Fig. 16(a) shows the entire construction of the sensor chip 400 in this embodiment. A sample dropping portion 401 onto which a test sample is dropped is provided on the sensor chip 400 at the end portion (on the right-hand side as viewed in Fig. 16(a)) side in the opposite insertion direction. A test sample dropped onto the sample dropping portion 401 is transferred through a sample inlet portion 402 to be introduced into a reaction reading portion 403, which is provided with a reagent for reading a reaction by such as a change in color. For example, the sample dropping portion 401 may be a recess in the sensor chip 3 surface, the sample inlet portion 402 may be a groove, and the reaction reading portion 403 may be paper such as filter paper stretched over a hole, or a space with a transparent window formed as a lower surface of the space. In this embodiment, the reaction portion is constituted by the sample dropping portion 401, the sample inlet portion 402, and the reaction reading portion 403. A hole 7 to be fitted around the retaining member 13 is provided in the sensor chip on the insertion side (on the left-hand side as viewed in the figure).

Fig. 16(b) shows the construction of essential portions of the analyzing device 404 and the construction of the sensor pack 2. A light source 405 and a light receiving portion 406 for optical reading are provided in the analyzing device 404 between the recess 191 in the bottom surface 111 of the opening 11 and the opening side of the analyzing device 404 at which the sensor pack 2 is inserted. The construction of the analyzing device 404 is the same as that in the first embodiment except that the light source 405 and the light receiving portion 406 are provided in place of the connection electrodes. The sensor pack 2 also has the same construction as that in the first embodiment. Also, the same method is used for setting the sensor chip 400. In this embodiment, the light source 405 and the light receiving portion 406 constitute a reaction information acquisition means.

Fig. 16(c) shows a state in which the retaining member 13 is fitted in the hole 7 of the sensor chip 400 to retain the sensor chip 400 and fitted to the recess 191. The sample dropping portion 401 of the sensor chip 404 is exposed outside the opening 11 and the reaction reading portion 403 is positioned above the light source 405 and the light receiving portion 406. In this state, a test sample is dropped onto the sample dropping portion 401 to enable measurement.

### (Fourteenth Embodiment)

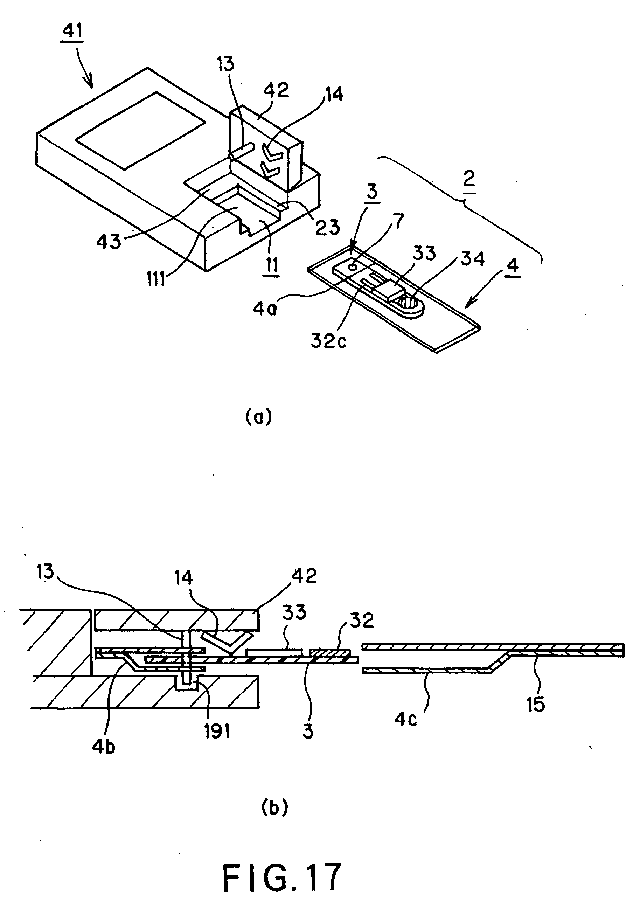

Figs. 17(a) shows a sensor chip, a sensor pack and an analyzing device in a fourteenth embodiment of the present invention. The same components or portions as those in the first and second embodiments are indicated by the same reference characters, and the description for them will be omitted.

The analyzing device 41 has the same construction as that in the second embodiment, and has a retaining member 13 and connecting electrodes 14 provided on a supporting member 42 which can be opened and closed by being moved relative to the main body of the analyzing device 41.

The packaging material 4 of the sensor pack 2 in this embodiment has generally the same structure as that in the first embodiment, but has a rupturing portion 4a provided in the lengthwise insertion direction by forming perforations or by half cutting, or the like. The rupturing portion 4a is positioned on the opposite side of the hole 7 of the end of the sensor chip 3 facing in the insertion direction, and is formed around the packaging material 4 along a shorter side direction of the packaging material 4. The portion of the base 6 corresponding to the hole 7 is formed so as to be penetrable by the retaining member 13.

The sensor pack 2 is placed on the bottom surface 111 of the opening 11 while being positioned with the guides portions 23,23 and the end stop portion 43, as in the second embodiment. The supporting member 42 is then closed. When the supporting member 42 is closed, the retaining member 13 tears the film 5, plunges through the hole 7 of the sensor chip 3 and further through the base 6 to reach the recess 191. At this time, the retaining member 13 retains not only the sensor chip 3 but also the packaging material 4. Therefore, when the packaging material 4 is pulled by holding the holdable extension 15, the packaging material 4 is ruptured at the rupturing process portion 4a as shown in Fig. 17(b). As a result, the packaging material 4 is separated in two to a device interior side portion 4b and a holdable extension 15 side portion 4c.

However, since the terminal electrode portions 32c of the sensor chip 3 are formed on the opening side of the rupturing process portion 4a and are not covered with the packaging material 4b, they can contact the connection electrodes 14. The reagent layer 34 is also located on the opening side of the rupturing portion 4a and is exposed outside the opening 11 of the analyzing device 41, so that a test sample can be supplied to the reagent layer 34.

After the measurement, the supporting member 42 is opened to enable the sensor chip 3 and the packaging material 4b to be thrown away. Since the sensor chip 3 and the package portion 4b are exposed when the supporting member 42 is opened, it is possible to throw away the sensor chip 3 and the package portion 4b by holding a suitable portion. In this manner, the sensor chip 3 can be thrown away without a risk of touching a test sample in a case where the test sample is a blood sample.

Thus, even when a portion of the packaging material is left in the analyzing device together with the sensor chip, in contrast with the case of removing the packaging material while retaining only the sensor chip as in the preceding embodiments, the sensor chip can be easily set in the analyzing device, and the risk of inadvertently touching the reagent layer at the time of setting of the sensor chip is reduced.

### (Fifteenth Embodiment)

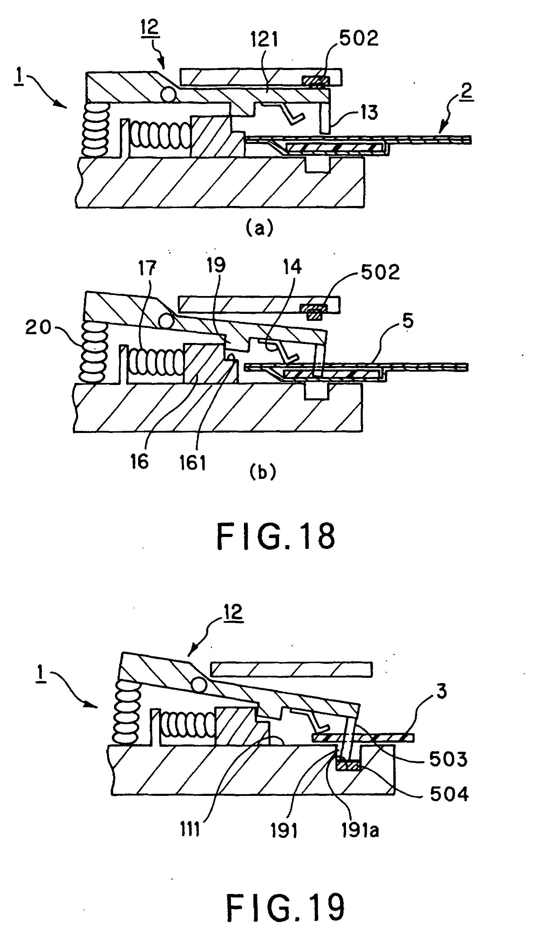

Figs. 18(a), 18(b), and 18(c) are cross-sectional views of essential portions of an analyzing device 1 and a sensor pack 2 constituting a sample ingredient analyzing system in a fifteenth embodiment of the present invention. These cross-sectional views are taken along a line passing through a center of the opening 11 in the lengthwise direction of the device to schematically show the construction of the essential portions of the analyzing device and the sensor pack. The same components or portions as those in the first embodiment are indicated by the same reference characters, and the description for them will be omitted.

In this embodiment, a switch 502 is provided in an inner surface of the frame of the analyzing device facing the arm portion. As shown in Fig. 18(a), before the sensor pack 2 is pushed in to move the slider 16, the projection 19 of the supporting member 12 is in contact with the upper surface of the slider 16, and the retaining member 13 is in a receding state. In this state, the switch 502 is inwardly displaced by the arm portion 121 of the supporting member 12, so that the power supply of the analyzing device 1 is off.

Next, as the sensor pack 2 in the state of contacting against the slider 16 is further inserted against the action of the spring 17, the projection 19 of the supporting member 12 falls to the stepped portion 161 of the slider 16, and the supporting member 12 rotates clockwise by the urging force of the spring 20, so that the retaining member 13 moves downward to tear the film 5, as shown in Fig. 18(b). At this time, the arm 121 moves away from the switch 502 and the power supply is turned on. The sensor chip 3 has the hole 7 formed so as to be engageable with the retaining member 13. The retaining member 13 moving downwards plunges through the hole 7 of the sensor chip 3 and stops by contacting against the base 6. At this time, the retaining member 13 is in the retaining state. However, the connection electrodes 14 have no electrical connection to the sensor chip 3 because of isolation by the film 5. In this embodiment, the supporting member 12, the spring 20, the slider 16 and the spring 17 constitute means for changing the state of the retaining means.

In this arrangement, the power supply is off before insertion of the sensor pack 2 and is turned on when the insertion of the sensor pack 2 to the predetermined position is completed. Therefore, it is not necessary for the operator to be conscious of the on/off state of the power supply. When the button 123 is pressed to enable drawing out of the sensor chip 3, the supporting member 12 is rotated counterclockwise and the arm portion 121 again presses the switch to turn off the power supply. That is, turning on/off of the power is effected through insertion and detachment of the sensor chip 3.

Fig. 19 shows an example of a modification of this embodiment.

The analyzing device 1 in this modification has the same construction as the above-described construction of this embodiment except for the construction of the switch. In this case, the retaining member 13 forms an electrode 503, and an electrode 504 is also formed on a bottom portion 191a of the recess 191 in the bottom surface 111, the electrode 504, facing the electrode 503. When the sensor chip 3 is retained, the sensor pack 2 is drawn out and the retaining member 13 plunges into the recess 191, thereby causing the electrodes 503 and 504 to contact each other to establish electrical connection therebetween. Through the electrical connection between the electrodes 503 and 504, the power supply is turned on. The button 123 is operated to return the supporting member 12 to the receding state to cut the electrical connection between the electrodes 503 and 504, as shown in Fig. 18(a), thereby turning off the power supply.

### (Sixteenth Embodiment)

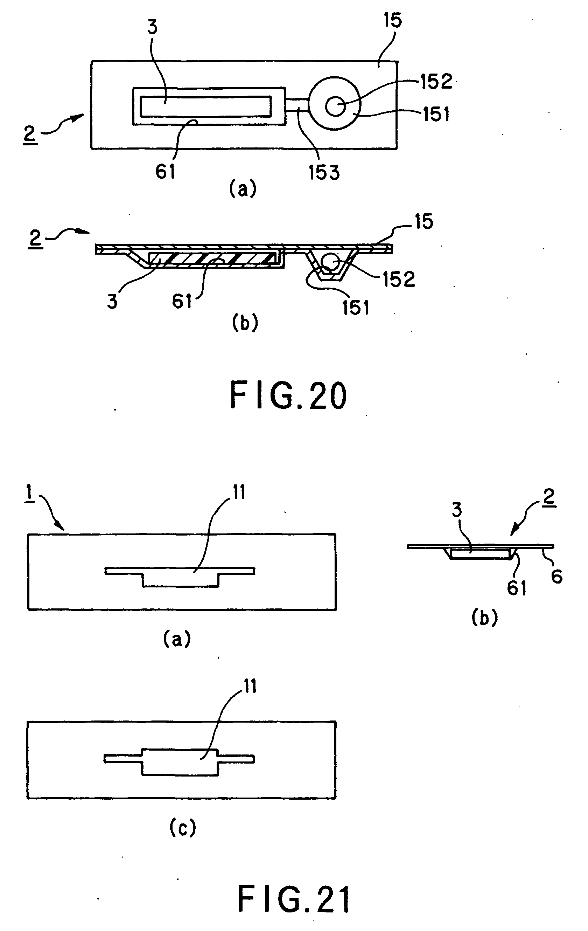

Figs. 20(a) and 20(b) are a top view and a cross-sectional view, respectively, of a sensor pack 2 constituting a sample ingredient analyzing system in a sixteenth embodiment of the present invention.

The sensor pack 2 is substantially the same as that in the first embodiment, and it will be described only with respect to different points. This sensor pack 2 can be used in the same analyzing device as that in the first embodiment.

The sensor pack 2 has a desiccant accommodation portion 151 in the form of a truncated cone formed by recessing the base 6 at the holdable extension 15 side. The desiccant accommodation portion 151 and the indented portion 61 are spaced apart from each other, but they are connected by channel for passage of air therebetween. In the desiccant accommodation portion 151, a spherical desiccant 152 is accommodated to dry air left in the recessed potion 61 when the sensor chip 3 is enclosed. The desiccant used in this sensor pack is, such as silica gel, activated alumina, synthetic zeolite, or magnesium. The shape of the desiccant is not limited to the spherical shape, and may have the shape of a rod or a sheet. Also, a method of providing a desiccant other than the method of accommodating an independent desiccant 152 in the desiccant accommodation portion 151 may be used. For example, the base or film may be formed of a resin material in which desiccant 152 is mixed. To form the concave and convex parts in the desiccant accommodation portion in the holdable extension 15, so that the sensor pack 2 can be held more securely. The provision of the desiccant accommodation portion 151 in the holdable extension 15 is advantageous in terms of the efficiency of space utilization and is effective in limiting the size of the sensor pack. Further, the channel functions as a reinforcement rib to increase the strength of the sensor pack 2.

### (Seventeenth Embodiment)

Figs. 21(a), 21(b), and 21(c) show a sensor pack 2 and an analyzing device 1 constituting a sample ingredient analyzing system in a seventeenth embodiment of the present invention.

Fig. 21(a) is a side view of the analyzing device 1 showing the end surface with opening 11 seen in the insertion direction of the sensor pack 2, and Fig. 21(b) is a side view of the sensor pack 2 showing the end surface seen in the insertion direction. The constructions of the analyzing device 1 and the sensor pack 2 are the same as those in the first embodiment except for the shape of the opening of the analyzing device 1, and description of the structure of internal portions, or the like of this embodiment will be omitted.

As shown in Fig. 21(a), the opening 11 is a generally T-shaped opening having a combination of the shape of a flat rectangle and the shape of another rectangle smaller in width and larger in height than the flat rectangle and adjacently located below a central portion of the flat rectangle. As shown in Fig. 21(b), the sensor pack 2 has a T-shaped side view formed by the flat base 6 and the indented portion 61.

That is, each of the opening 11 and the sensor pack 2 has an asymmetrical shape about a horizontal line corresponding to the surface of the sensor chip 3. Therefore, the sensor pack 2 cannot be inserted into the opening 11 when positioned top side down. The reagent layer 34 and the terminal electrode portions 32c of the sensor chip 3 are provided on the upper surface side, and the setting orientation of the sensor chip 3 is prescribed (see Fig. 1). For this reason, if it is set top side down to the surface, measurement cannot be carried out. However, if the opening 11 and the sensor pack 2 are made asymmetrical about a horizontal line, there is no possibility of failure to correctly set the sensor pack insertion direction.

While the sensor pack 2 is shaped as shown in Fig. 21(b), the opening 11 of the analyzing device 1 may alternatively have a crisscross shape such as shown in Fig. 21(c). In such a case, the height of the top rectangular section of the crisscross opening 11 is reduced relative to the depth of the indented portion 61 of the sensor pack 2, and the height of the bottom rectangular section of the opening 11 is increased relative to the depth of the indented portion 61 of the sensor pack 2. In this manner, the opening 11 can formed so as to be asymmetrical about a horizontal line, thereby preventing the sensor pack 2 from being inserted into the opening 11 in the state of being turned top side down.

### (Eighteenth Embodiment)

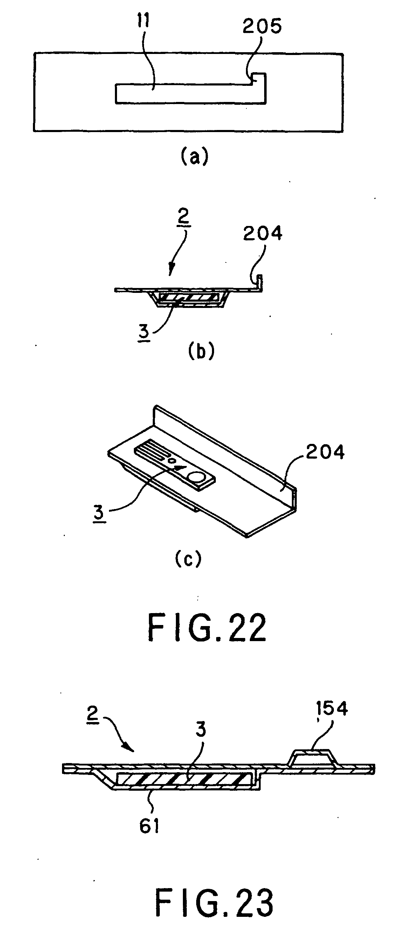

Figs. 22(a), 22(b), and 22(c) show a sensor pack 2 and an analyzing device 1 constituting a sample ingredient analyzing system in an eighteenth embodiment of the present invention.

Fig. 22(a) is a side view of the analyzing device 1 showing the end surface with the opening 11 seen in the insertion direction of the sensor pack 2, and Fig. 22(b) is a side view of the sensor pack 2 showing the end surface seen in the insertion direction. The constructions of the analyzing device 1 and the sensor pack 2 are the same as those in the first embodiment except for the shape of the opening of the analyzing device 1 and the shape of the base 6 of the sensor pack, and description of the structure of the internal portions, and the like of this embodiment will be omitted.

As shown in Fig. 22(a), the opening 11 has a rectangular shape elongated in the horizontal direction and having a bent portion 115 upwardly bent at the right end portion. The height of the rectangular portion is larger than the depth of the indented portion 61 of the sensor pack 2.

As shown in Fig. 22(b), the sensor pack 2 has a bent portion 205 upwardly bent at the right end portion as seen in the insertion direction. This bent portion 205 is fitted to the bent portion 115 of the opening 11.

That is, each of the opening 11 and the sensor pack 2 is asymmetrical about a line perpendicular to the surface of the sensor chip 3. Therefore, the sensor pack 2 cannot be inserted into the opening 11 when positioned top side down or positioned to insert the wrong end. Thus, if the opening 11 and the sensor pack 2 are made asymmetrical about a vertical line, there is no possibility of failure to correctly set the sensor pack 2 to in the insertion direction.

### (Nineteenth Embodiment)

Fig. 23 shows a sensor pack constituting a sample ingredient analyzing system in a nineteenth embodiment of the present invention. The same analyzing device as that in the first embodiment can also be used in this embodiment, and the description for it will be omitted.

Fig. 23 is a longitudinal cross-sectional view of the sensor pack 2 of this embodiment. The sensor pack 2 is the same as that in the first embodiment except for the holdable extension 15. In this embodiment, a projection 154 is formed on the upper surface side of the holdable extension 15 so that the height of the projection 154 is larger than the depth of the indented portion 61. In this manner, an insertion-side portion of the sensor pack 2 and another portion of the sensor pack 2 opposite from the insertion-side portion can have different shapes, such that the wrong end of the sensor pack 2 cannot be inserted into the opening 11. In a case where a desiccant accommodation portion 151 is provided in the holdable extension 15 portion as shown in Fig. 20(a), the depth of the desiccant accommodation portion 151 may be increased relative to the depth of the indented portion 61, so a shape at the insertion side and a shape at the opposite side in the sensor pack 2 is different from each other, so that the wrong end of the sensor pack 3 cannot be inserted into the opening 11.

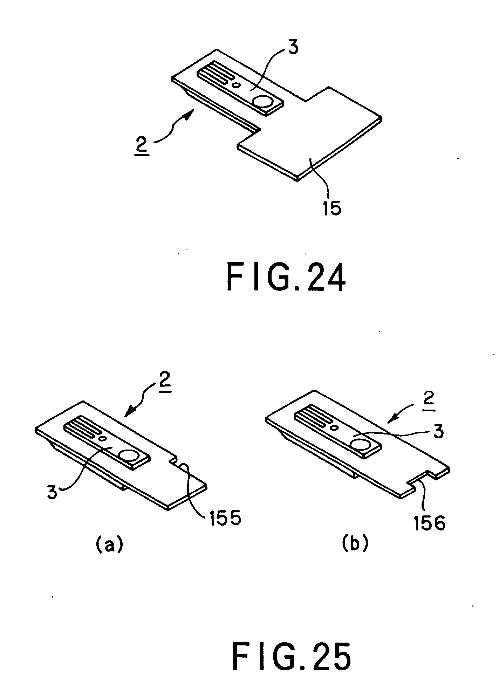

Fig. 24 is a schematic perspective view of a first example of modification of this embodiment. The width of the holdable extension 15 is increased relative to the width on the insertion side. In this manner, a shape at the insertion side and a shape at the opposite side in the sensor pack 2 can be made different from each other, so that the wrong end of the sensor pack 3 cannot be inserted into the opening 11.

Fig. 25 is a schematic perspective view of a second modification example of this embodiment. One side portion of the holdable extension 15 is cut along the lengthwise direction to form a cut portion 155 (Fig. 25(a)), or the end portion is cut to form a cut portion 156 (Fig. 25(b)). In a case where the analyzing device is arranged so that when the sensor pack 3 is inserted, a switch in the device is operated by a portion of the sensor pack 3, the portion corresponding to the switch when the end on the holdable extension 15 side is inserted may be cut to prevent the switch from being operated by insertion of the wrong end into the opening 11. If the analyzing device and the sensor pack are arranged in this manner, measurement is not started even if the wrong end is inserted, and the inserting operation of the correct end can then be performed to use the sensor pack.

### (Twentieth Embodiment)

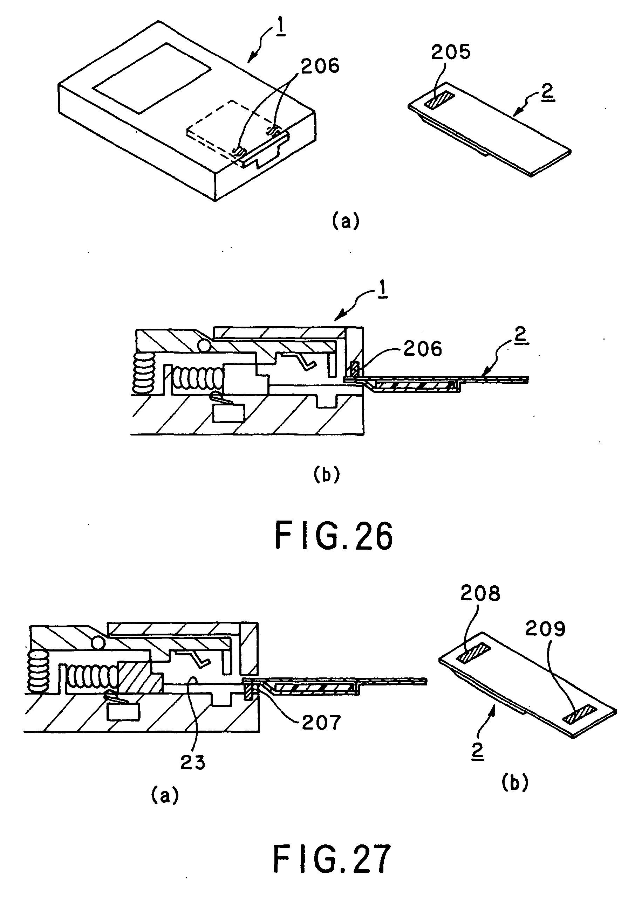

Fig. 26(a) is a perspective view of an analyzing device 1 and a sensor pack 3 in a twentieth embodiment of the present invention. The constructions of essential portions of the analyzing device 1 and the sensor pack 3 are the same as those in the first embodiment, and this embodiment will be described with respect to different points.

As shown in Fig. 26(a), a conductive portion 205 (a portion to be detected) is provided in the upper surface of a tip end portion of the sensor pack 3 at the in the insertion direction, and two electrodes 206 (detecting portions) are provided in a lower surface of an upper frame member of the analyzing device 1. When the sensor pack 3 is inserted in the normally oriented state, the upper surface of the sensor pack 2 and the lower surface of the upper frame member of the analyzing device 1 face each other. In this case, therefore, when the tip end of the sensor pack 3 is inserted into the opening 11, the electrodes 206,206 and the conductive portion 205 contact each other to establish an electrical connection between the electrodes 206, as shown in Fig. 26(b). On the other hand, when the sensor pack 3 is inserted in a wrong oriented directed, no electrical connection is established between the two electrodes 206, thus enabling ascertainment as to whether the sensor pack 3 is inserted in the correctly oriented state or in a wrong oriented state.

The conductive portion 206 may be formed by applying a conductive material to the surface of the film 6. If the film 6 is a film of a metal material, such as aluminum the portion of the film surface other than the portion for the conductive portion may be covered with a nonconductive material such as a resin by laminating or the like.

Such results of detection of the inserted states of the sensor pack may be utilized to automatically turn on the power supply or to automatically start a measurement preparation process.

In the above-described embodiment, the electrodes and the conductive portion are electrically connected when the sensor pack 3 is inserted in the normally oriented state. It is possible that, alternatively, the electrodes and the conductive portion are electrically connected when the sensor pack 2 is inserted in a wrong oriented state.

Figs. 27(a) and 27(b) show an example of such a modification. The constructions of essential portions of an analyzing device 1 and a sensor chip 3 are the same as those in the first embodiment. Electrodes 207 are provided on guides portions 23 , as shown in Fig. 27(a), and conductive portions 208 and 209 are respectively provided on an upper surface portion close to the tip end in the insertion direction and the upper surface portion in the opposite end, as shown in Fig. 27(b). In this arrangement, when the sensor pack 2 is inserted in the normally oriented state as shown in Fig. 27(a), no electrical connection is established between the electrodes 207. When the sensor pack is inserted in the state of being turned top side down or the wrong end is inserted, the electrodes 207 and the conductive portion 208 or 209 are electrically connected to each other. Thus, it is possible to ascertain whether the sensor chip 2 is inserted in the normally oriented state or in a wrong oriented state.

In this embodiment, the electrodes and the conductive portion constitute inserted state detection means. However, optical detection means such as a photointerruptor may alternatively be used. Also, a portion to be detected may be formed by marking a color and the color may be detected by a detecting portion.

### (Twenty-first Embodiment)

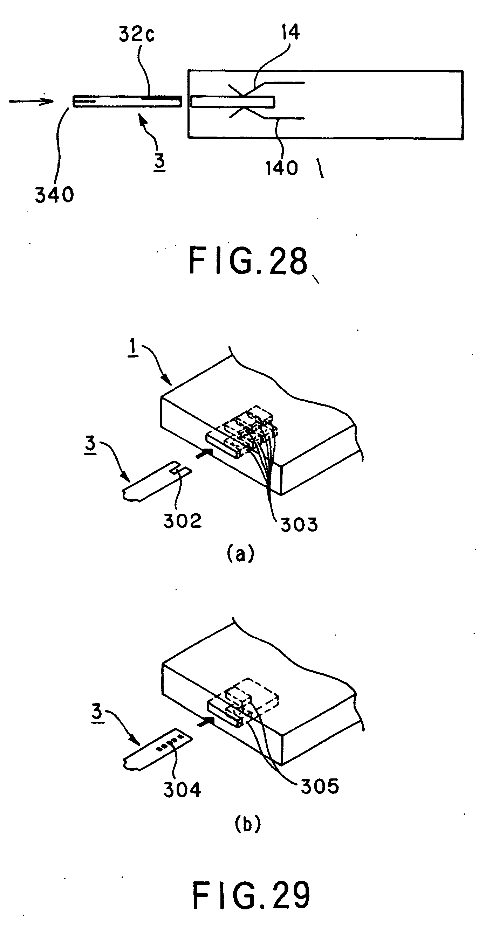

In Fig. 28, illustration of the structure of the base and film is omitted for convenience of explanation. The constructions of the essential portions of an analyzing device 1 and a sensor chip 3 are the same as those in the first embodiment. The same components or portions as those in the first embodiment are indicated by the same reference characters, and the description for them will be omitted.

In this embodiment, a pair of connection electrodes 14 and a pair of connection electrodes 140 constituting reaction information acquisition means are respectively provided on the lower surface of an upper portion frame member of the analyzing device facing the upper surface ot the sensor chip 3 and the upper surface of a lower portion frame member of the analyzing device facing the lower surface of the sensor chip 3. Ordinarily, terminal electrode portions 32c of the sensor chip 3 are provided at only one side, and only one pair of connection electrodes capable of establishing an electrical connection only at the time of insertion of the sensor pack in the normally oriented state are provided. However, in a situation where a sample supply port 340 is formed in an end surface facing in the lengthwise direction, or in a like situation, a measurement can be carried out without a problem even if the sensor chip 2 is set in a state of being turned top side down. Even in a case where reagent layer 34 is formed on one of the surfaces, a measurement can be carried out by reversing the analyzing device if two pairs of connection electrodes are provided. Therefore, if connection electrodes are provided on the opposite sides of the surface of the sensor chip 3 as described above, it is possible for the operator to insert the sensor pack 2 without paying attention to setting of the correct side.

### (Twenty-second Embodiment)

Fig. 29(a) schematically shows the construction of related portions of an analyzing device 1 and a sensor chip 3 constituting a sample ingredient analyzing system in a twenty-second embodiment of the present invention. The constructions of the essential portions of the analyzing device 1 and the sensor chip 3 are the same as those in the first embodiment, and the description for them will be omitted. In Fig. 29(a), illustration of the construction of the sensor pack is omitted for convenience of explanation.

In this embodiment, a cut portion (information holding means) 302 of a predetermined size is provided at the end of the sensor chip 3 in the insertion direction, and the size of the cut portion 302 is detected by photosensors (information recognition means) 303 provided in the analyzing device 1 so as to face the inner space of the opening 11. The size of the cut portion 302 may be set according to information such as a lot number, a correction value, the data of manufacture, of the sensor chip 3. Such information can be recognized and input through the photosensors 303. If such means are used, it is possible to eliminate the need for a preliminary setting process including inserting a correction chip in the analyzing device and inputting a correction value. This arrangement is also effective in preventing such as occurrence of an input error and omission of correction.

Fig. 29(b) shows an example of a modification of this embodiment. Holes 304 are formed in an end portion of the sensor chip 3 in the insertion direction. The number of holes 304, the pitch between holes 304, and the like are detected by photosensors 305 on the analyzing device side. Also in this case, the number of holes 304, the pitch, and the like may be set according to information relating to correction to obtain the same effects.

### (Twenty-third Embodiment)

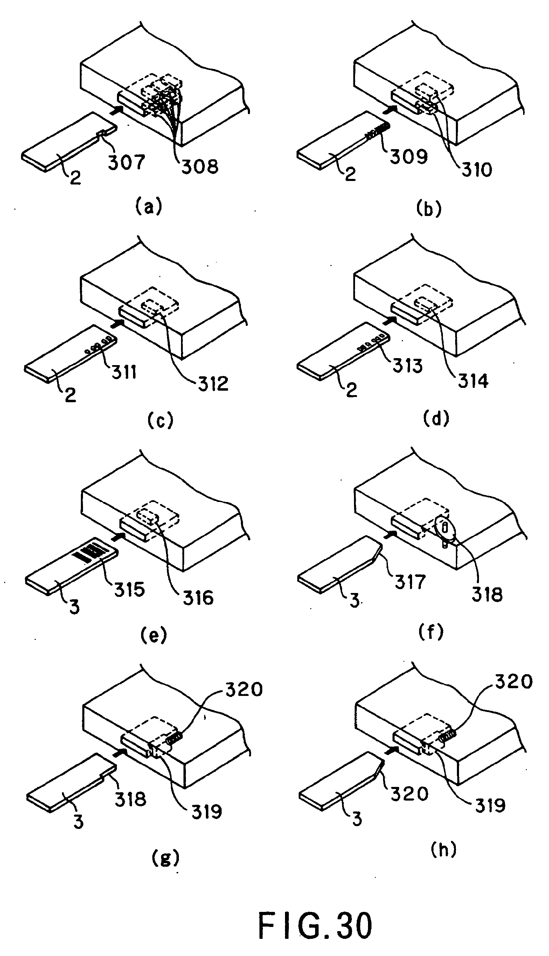

Figs. 30(a) to 30(h) schematically show the construction of related portions of an analyzing device 1 and a sensor pack 2 constituting a sample ingredient analyzing system in a twenty-third embodiment of the present invention. The constructions of essential portions of the analyzing device 1 and the sensor chip 3 are the same as those in the first embodiment, and description for them will be omitted.

In this embodiment, the same arrangement as that in the twenty-second embodiment is realized by the analyzing device 1 and the sensor pack 2.

Fig. 30(a) shows a case where a cut portion (information holding means) 307 of a predetermined size is provided in a side edge of the sensor pack 2, and the size of the cut portion 307 is detected by photosensors (information recognition means) 308 provided in the analyzing device 1 so as to face the inner space of the opening 11. Also, the size of the cut portion 307 may be set according to information on such as a lot number, a correction value, the data of manufacture of the sensor chip 3. This information can be recognized and input through photosensors 308. If such means are used, it is possible to eliminate the need for a preliminary setting process including inserting a correction chip into the analyzing device and inputting a correction value. This arrangement is also effective in preventing such as occurrence of an input error and omission of correction.

Fig. 30(b) shows a case where a predetermined number of cut portions (information holding means) 309 having a predetermined size and pitch are provided in a side edge portion of the sensor pack 2, and the cut portions 309 are detected by photosensors (information recognition means) 310 provided in the analyzing device 1 so as to face the inside of the opening 11. The size of cut portions 309 may be set according to information on such as a lot number, a correction value, or the data of manufacture of the sensor chip 3. This information can be recognized and input through photosensors.

Fig. 30(c) shows a case where a predetermined number of projections (information holding means) 311 having a predetermined pitch are provided on a side edge portion of the sensor pack 2, and a switch (information recognition means) 312 facing the inner space of the opening 11 is operated by projections 311 when the sensor pack 2 is inserted. The number and the pitch of projections 311 may be set according to information on such as a lot number, a correction value, or the data of manufacture of the sensor chip 3. This information can be recognized and input through the switch 312.

Fig. 30(d) shows a case where a predetermined number of recesses (information holding means) 313 having a predetermined pitch are provided in a side edge portion of the sensor pack 2, and a switch (information recognition means) 314 facing the inner space of the opening 11 is operated by recesses 313 when the sensor pack 2 is inserted. The number and the pitch of recesses 313 may be set according to information on such as a lot number, a correction value, or the data of manufacture of the sensor chip 3. This information can be recognized and input through the switch 314.

Fig. 30(e) shows a case where a bar-code pattern (information holding means) 315 is printed on the sensor pack 2, and this pattern 315 is read in by a photosensor (information recognition means) 316 facing the inner space of the opening 11 when the sensor pack 2 is inserted. If information of a lot number, a correction value, the data of manufacture and the like of the sensor chip 3 is converted into a bar-code pattern and printed, this information can be recognized and input through the photosensor 316.

Fig. 30(f) shows a case where one corner of the end of the sensor pack 2 to be inserted is cut to form a slanting surface (information holding means) 317, and a cam 318 projecting into the inner space of the opening 11 of the analyzing device 1 is rotated by the slanted surface 317. The cam 318 is rotatably amounted about a predetermined shaft. The distance from the center of rotation to the peripheral surface of the cam 318 changes with respect to the angle of rotation of the cam 318. When the sensor chip 2 is inserted through the opening 11, the slanting surface 317 is brought into contact with the peripheral surface of the cam 318, and the cam 318 rotates according to the angle of the slanting surface 317. Therefore, from the angle of this rotation, the angle of the slanting surface 317 can be detected. The angle of the slanting surface 317 may be set according to information on such as a lot number, a correction value, or the data of manufacture, of the sensor chip 3. This information can be recognized and input through a rotational angle detection means of the cam 318. The cam 318 and the rotational angle detection means constitute an information recognition means.

Fig. 30(g) shows a case where a side edge portion of the tip end of the sensor pack 2 to be inserted is cut to form a stepped portion (information holding means) 318, and a moving member 319 provided inside the opening of the analyzing device 1 is pushed in by the stepped portion 318. The moving member 319 is movable along the direction of insertion of the sensor pack 2 and is urged by a spring toward the opening 11. The moving member 319 moves according to the position of the stepped portion 318 of the sensor pack 2, and the amount of movement of the moving member 319 can be detected. The position of the stepped portion 318 may be set according to information on some of a lot number, a correction value, the data of manufacture, etc., of the sensor chip 3. This information can be recognized and input through means for detecting the amount of movement of the moving member 319. As shown in Fig. 30(h), one corner of the end of the sensor pack 2 to be inserted is cut to form a slanting surface (information holding means) 320. The amount of movement of the moving portion is determined by the angle of the slanting surface. Therefore, if the angle of the slanting surface is set according to information on such as a lot number, a correction value, or the data of manufacture of the sensor chip 3. This information can also be recognized and input through the means for detecting the amount of movement of the moving member. The moving portion 319 and the moving amount detection means constitute an information recognition means.

The information recognition means in this embodiment can also be used as insertion direction determination means for making a determination as to whether the orientation of the sensor pack or the sensor chip with respect to the insertion direction is correct by checking whether information from the information holding means can be recognized.

### (Twenty-fourth Embodiment)

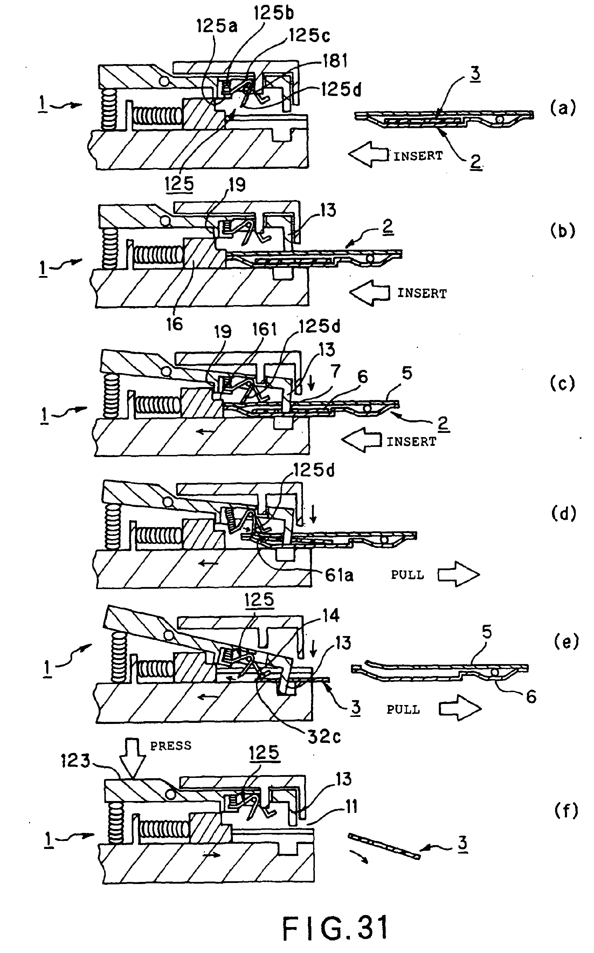

Figs. 31(a) to 31(f) are longitudinal sectional views of an analyzing device 1 and a sensor pack 2 constituting a sample ingredient analyzing system in a twenty-fourth embodiment of the present invention.

The construction of essential portions of the analyzing device 1 is the same as that in the first embodiment, and the construction of the sensor pack 2 is the same as that in the sixteenth embodiment of the present invention. The same components or portions as in those embodiments are indicated by the same reference characters, and the description for them will be omitted.

In this embodiment, as shown in Fig. 31(a), a cutter (opening forming means) 125 is provided on a lower surface of the supporting member 12. The cutter 125 is V-shaped, has a base portion 125a connected to the lower surface of the supporting member 12 through a spring 125b, and has a bent portion 125c downwardly bent, an inner surface of which slidably contacts a shaft 181 mounted parallel to the shaft 18 of the supporting member 12. The cutter 125 also has a blade 125d at its end portion opposite from the base portion 125a extending along an axial direction parallel. When the sensor pack 2 is inserted and the supporting member 12 is in the state of receding to the upper surface side, the blade 125d of the cutter 125 is also in a receding state and does not interfere with the sensor pack 2 at the time of insertion (Fig. 31(b)). As the sensor pack 2 is further pushed in after being brought to the position of contacting against the slider 16, the projection 19 of the supporting member 12 falls to the stepped portion 161 of the slider 16 and the supporting member 12 rotates clockwise about the shaft 18 (Fig. 31(c)). At this time, the retaining member 13 plunges through the hole 7 of the sensor chip 3 and stops by contacting the base 6, and the blade 125d slightly bites into the film 5 in a direction opposite to the insertion direction at a position corresponding to the top of the sloping surface 61a of the indented portion 61. Thereafter, when the sensor pack 2 is pulled, the blade 125d further bites into the film 5 and forms an opening in the film 5 by tearing the same. With the further movement of the sensor pack 2, the blade 125d moves on the sloping surface 61a and therefore the cutter 125 rotates counterclockwise (Fig. 31(d)). At this time, the spring 125b is stretched and the base potion 125a is urged clockwise by the urging force of the spring 125b, so that the blade 125d further bites toward the sloping surface 61a to widen the opening. Further, when the base 6 and the film 5 are drawn out, the sensor chip 3 is securely retained by the retaining member 13, and the connection electrodes 14 are electrically connected to the terminal electrode portions 32c (Fig. 31(e)). Thereafter, when the button 123 is pressed, the supporting member 12 rotates counterclockwise, the slider 16 moves toward the opening 11, and the retaining member 13 is disengaged from the retaining state, thereby allowing the sensor chip 3 to be thrown away from the opening 11 (Fig. 31(f)).

In this arrangement, it is not necessary to tear the film 5 by the tip end of the sensor chip 3, there is no feeling of resistance when the sensor chip 3 is taken out, and taking out and setting of the sensor chip can be completed without requiring a substantially large force.

### (Twenty-fifth Embodiment)

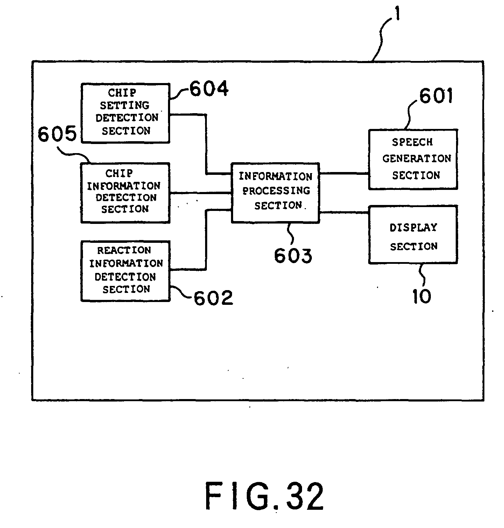

Fig. 32 shows a block diagram of an analyzing device 1 constituting a sample ingredient analyzing system in a twenty-fifth embodiment of the present invention.

In this embodiment, the analyzing device 1 has a speech generation section (speech generation means) 601 such as a speaker. A reaction information detection section 602 is constituted by the connection electrodes 14 and signal processing means such as an amplifier and an analog to digital (A/D) converter. The reaction information detection section 602 detects information on a reaction on the sensor chip 3. An information processing section 603 performs ingredient analysis processing, such as computation of the concentration of a particular ingredient in a test sample on the basis of data sent from the reaction information detection section 602. Data from a chip setting information detection section 604 constituted by a detection section and means for processing a signal from the detection section, and data from a chip information detection section 605 constituted by photosensors or the like and means for processing signals from the photosensors are also supplied to the information processing section 603. The speech generation section 601 and the display section 10 are connected to the information processing section 603. On the basis of data and commands supplied from the information processing section 603, the speech generation section 601 or the display section 10 informs the operator of the results of a measurement, performs guidance on the process of setting the sensor chip or the measuring process, and outputs a speech for information on an error or a remeasurement instruction or provides a visual display of such information using characters, symbols, and figures. Because the speech generation section 601 is provided, even a weak-sighted person can easily operate the system and can easily understand the results of the operation.

## Claims

1. A sample ingredient analyzing system comprising:
a sensor pack formed by packing in a packaging material a sensor chip having a reaction portion for reacting with a test sample; and
an analyzing device having an opening for accepting said sensor pack containing one sensor chip, and retaining means for retaining the sensor chip in the sensor pack accepted through the opening, said analyzing device analyzing an ingredient in a test sample supplied to the reaction portion by detecting a change in the reaction portion.

2. A sample ingredient analyzing system according to Claim 1, wherein said sensor chip has engagement means for engagement with said retaining means.

3. A sample ingredient analyzing system according to Claim 1, wherein said retaining means penetrates said packaging material at least to reach said sensor chip.

4. A sample ingredient analyzing system according to Claim 1, wherein said sensor pack has a holding to be held by a user.

5. A sample ingredient analyzing system according to Claim 1, wherein said packaging material has positioning means for positioning the sensor chip.

6. A sample ingredient analyzing system according to Claim 1, wherein said analyzing device has positioning means for positioning the sensor chip when only the sensor chip is inserted through said opening.

7. A sample ingredient analyzing system according to Claim 1, wherein said analyzing device has retention undoing means for undoing the retention continued by said retaining means.

8. A sample ingredient analyzing system according to Claim 1, wherein said analyzing device has state changing means for changing the state of said retaining means between a state of receding from said sensor pack and a state of retaining said sensor chip, and
wherein said state changing means changes the state of said retaining means so that said retaining means is in the receding state when said sensor pack is inserted, and so that said retaining means is in the retaining state after the completion of insertion of the sensor pack.

9. A sample ingredient analyzing system according to Claim 8, wherein said state changing means is operated by a movable member which is moved by insertion of the sensor pack.

10. A sample ingredient analyzing system according to Claim 9, wherein said analyzing device has a power supply switch operated by the movement of said movable member.

11. A sample ingredient analyzing system according to Claim 8, wherein said analyzing device has a power supply switch capable of turning on and off the power supply to said analyzing device by being linked to the two states of said retaining means.

12. A sample ingredient analyzing system according to Claim 1, wherein said analyzing device has reaction information acquisition means for obtaining information on reaction at the reaction portion from said sensor chip, and
wherein said analyzing device positions said reaction information acquisition means on said sensor chip by retaining said sensor chip by said retaining means.

13. A sample ingredient analyzing system according to Claim 1, wherein, when said packaging material is removed from said opening while said sensor chip is retained by said retaining means, said sensor chip is taken out from said packaging material in such a manner that said sensor chip is brought into contact with said packaging material to tear said packaging material, and
wherein said packaging material has a force receiving portion provided at a position at which said sensor chip is brought into contact with said packaging material, a force applied by said sensor chip being concentrated at said force receiving portion.

14. A sample ingredient analyzing system according to Claim 13, wherein a portion of said sensor chip remote from said reaction portion is taken out first from the packaging material.

15. A sample ingredient analyzing system according to Claim 1, wherein said analyzing device has reaction information acquisition means for obtaining information on reaction at the reaction portion from said sensor chip, said reaction information acquisition means being provided in said retaining means.

16. A sample ingredient analyzing system according to Claim 3, wherein said packaging material has a penetrable portion through which said retaining means can penetrate, and a penetration stop portion which stops the penetrating action of said retaining means,
wherein said analyzing device has:
reaction information acquisition means for obtaining information on reaction at said reaction portion by being brought into contact with said sensor chip; and
state changing means for changing the state of said reaction information acquisition means between a first state of being spaced apart from said sensor pack or loosely contacting said sensor pack and a second state of contacting said sensor chip, and
wherein said state changing means sets said reaction information acquisition means in said first state when said retaining means penetrates said penetrable portion to retain said sensor chip, and sets said reaction information acquisition means in said second state when said packaging material is removed from said opening, and when said retaining means is retaining only said sensor chip.

17. A sample ingredient analyzing system according to Claim 1, wherein said sensor pack contains a desiccant.

18. A sample ingredient analyzing system according to Claim 17, wherein said sensor pack has a holding to be held by a user, and a desiccant accommodation portion for accommodating the desiccant is provided in said holding.

19. A sample ingredient analyzing system according to Claim 1, wherein a predetermined orientation of said sensor pack with respect to the direction of insertion into the opening of said analyzing device is prescribed, and
wherein a cross-sectional shape of said sensor pack as viewed in the direction of insertion when said sensor pack has an orientation different from said predetermined orientation is different from a cross-sectional shape of said opening as viewed in the direction of insertion.

20. A sample ingredient analyzing system according to Claim 19, wherein said sensor chip has the shape of a generally flat block, and each of said sensor pack and said opening has a shape exhibiting an asymmetry on the opposite sides of the two surfaces of the sensor chip.

21. A sample ingredient analyzing system according to Claim 19, wherein said sensor chip has the shape of a generally flat block, and each of said sensor pack and said opening has a shape asymmetric as seen in opposite directions along a surface of the sensor chip.

22. A sample ingredient analyzing system according to Claim 1, wherein a predetermined orientation of said sensor chip with respect to the direction of insertion into the opening of said analyzing device is prescribed, and
wherein a portion of said sensor pack on one side in the direction of insertion along said predetermined orientation and another portion of said sensor pack on the opposite orientation side differ in shape from each other.

23. A sample ingredient analyzing system according to Claim 1, further comprising inserted state detection means for detecting an insertion state of the sensor pack having a detecting portion provided in said analyzing device and a portion to be detected provided in said sensor pack at a predetermined position.

24. A sample ingredient analyzing system according to Claim 1, wherein said analyzing device has first reaction information acquisition means for obtaining information on reaction at said reaction portion from said sensor chip when said sensor chip is inserted in the state of having said predetermined orientation to the opening, and second reaction information acquisition means for obtaining information on reaction at said reaction portion from said sensor chip when said sensor chip is inserted in the opening in the state of having an orientation different from said predetermined orientation.

25. A sample ingredient analyzing system according to Claim 1, further comprising information holding means for holding information on said sensor chip, said information holding means being provided on at least one of said sensor pack and said sensor chip, and information recognition means for recognizing information held by said information holding means, information recognition means being provided in said analyzing device.

26. A sample ingredient analyzing system according to Claim 25, further comprising insertion orientation determination means for making a determination as to whether the orientation of said sensor chip with respect to the direction of insertion is correct by checking whether information from said information holding means can be recognized by said information recognition means.

27. A sample ingredient analyzing system according to Claim 1, wherein said analyzing device has opening forming means for forming an opening in the packaging material of said sensor pack.

28. A sample ingredient analyzing system according to Claim 1, wherein said analyzing device has speech generation means.

29. A sensor chip for use in a sample ingredient analyzing system having a sensor pack formed by packing in a packaging material a sensor chip having a reaction portion for reacting with a test sample, and an analyzing device having an opening for accepting the sensor pack containing one sensor chip, and retaining means for retaining the sensor chip in the sensor pack accepted through the opening, the analyzing device analyzing an ingredient in a test sample supplied to the reaction portion by detecting a change in the reaction portion, said sensor chip comprising engagement means for engagement with the retaining means of the analyzing device.

30. A sensor pack for use in a sample ingredient analyzing system having said sensor pack formed by packing in a packaging material a sensor chip having a reaction portion for reacting with a test sample, and an analyzing device for analyzing an ingredient in a test sample supplied to the reaction portion by detecting a change in the reaction portion, said sensor pack comprising a holding to be held by a user.

31. A sensor pack for use in a sample ingredient analyzing system having said sensor pack formed by packing in a packaging material a sensor chip having a reaction portion for reacting with a test sample, and an analyzing device for analyzing an ingredient in a test sample supplied to the reaction portion by detecting a change in the reaction portion, said sensor pack comprising positioning means for positioning the sensor chip in the packaging material.

32. A sensor pack for use in a sample ingredient analyzing system having said sensor pack formed by packing in a packaging material a sensor chip having a reaction portion for reacting with a test sample, and an analyzing device for analyzing an ingredient in a test sample supplied to the reaction portion by detecting a change in the reaction portion, in which system, when the packaging material is removed from an opening of the analyzing device while the sensor chip is retained by the retaining means, the sensor chip is taken out from the packaging material in such a manner that the sensor chip is brought into contact with the packaging material to tear the packaging material, said sensor pack comprising a force receiving portion provided in the packaging material at a portion at which the sensor chip is brought into contact with the packaging material, where a force applied by the sensor chip is concentrated.

33. A sensor pack for use in a sample ingredient analyzing system having said sensor pack formed by packing in a packaging material a sensor chip having a reaction portion for reacting with a test sample, and an analyzing device for analyzing an ingredient in a test sample supplied to the reaction portion by detecting a change in the reaction portion, said sensor pack comprising a desiccant.

34. A sensor pack according to Claim 33 having a holding to be held by a user, wherein a desiccant accommodation portion for accommodating the desiccant is provided in said holding.

35. A sensor pack for use in a sample ingredient analyzing system having said sensor pack formed by packing in a packaging material a sensor chip having a reaction portion for reacting with a test sample, and an analyzing device for analyzing an ingredient in a test sample supplied to the reaction portion by detecting a change in the reaction portion, said sensor pack comprising:
a predetermined orientation of said sensor pack being prescribed with respect to the direction of insertion into an opening of the analyzing device; and
a cross-sectional shape of said sensor pack as viewed in the direction of insertion when said sensor pack has an orientation different from said predetermined orientation being different from a cross-sectional shape of the opening of the analyzing device as viewed in the direction of insertion of the sensor pack.

36. A sensor pack for use in a sample ingredient analyzing system having said sensor pack formed by packing in a packaging material a sensor chip having a reaction portion for reacting with a test sample, and an analyzing device for analyzing an ingredient in a test sample supplied to the reaction portion by detecting a change in the reaction portion, said sensor pack comprising:
a portion of the sensor pack on one side in the direction of insertion and another portion of the sensor pack on the opposite side differing in shape from each other.

37. A sensor pack for use in a sample ingredient analyzing system having said sensor pack formed by packing in a packaging material a sensor chip having a reaction portion for reacting with a test sample, and an analyzing device for analyzing an ingredient in a test sample supplied to the reaction portion by detecting a change in the reaction portion, said analyzing device having information recognition means, said sensor pack comprising:
information holding means for holding information recognizable by said information recognition means.
